# EUROPEAN PATENT SPECIFICATION

(11) **EP 1 694 849 B1**
(45) Date of publication and mention of the grant of the patent: **14.09.2011**
(21) Application number: 04816347.1
(22) Date of filing: 17.12.2004
(51) Int. Cl.: C12N 15/82, A01H 5/00, C07K 14/415

(54) **PLANTS HAVING MODIFIED GROWTH CHARACTERISTICS AND METHOD FOR MAKING THE SAME**
PFLANZEN MIT MODIFIZIERTEN WACHSTUMSEIGENSCHAFTEN SOWIE HERSTELLUNGSVERFAHREN DAFÜR
VEGETAUX A CARACTERISTIQUES DE CROISSANCE MODIFIEES ET LEURS METHODES D'ELABORATION

(30) Priority: 17.12.2003 EP 03104764; 22.12.2003 US 531866 P
(43) Date of publication of application: 30.08.2006
(73) Proprietor: CropDesign N.V., 9052 Zwijnaarde (BE)
(72) Inventor: FRANKARD, Valerie, B-1640 Sint-Genesisus-Rode (BE); MIRONOV, Vladimir, B-9000 Gent (BE); SANZ MOLINERO, Ana Isabel, B-9050 Gentbrugge (BE)
(74) Representative: Mistry, Meeta
(86) International application number: PCT/EP2004/053594
(87) International publication number: WO 2005/059147

(56) References cited:
- WO-A-03/085115
- DOERKS TOBIAS ET AL: "Systematic identification of novel protein domain families associated with nuclear functions" GENOME RESEARCH, vol. 12, no. 1, January 2002 (2002-01), pages 47-56, XP002283274 ISSN: 1088-9051 cited in the application
- DATABASE TREMBL [Online] 1 May 1999 (1999-05-01), XP002283275 retrieved from EBI Database accession no. Q9ZU93
- PENG JINRONG ET AL: "'Green revolution' genes encode mutant gibberellin response modulators" NATURE (LONDON), vol. 400, no. 6741, 15 July 1999 (1999-07-15), pages 256-261, XP002283524 ISSN: 0028-0836
- RANCOUR DAVID M ET AL: "Plant UBX domain-containing protein 1, PUX1, regulates the oligomeric structure and activity of arabidopsis CDC48." JOURNAL OF BIOLOGICAL CHEMISTRY, ELECTRONIC PUBLICATION: 2004-10-21. JOURNAL CODE: 2985121R. ISSN: 0021-9258., vol. 279, no. 52, 24 December 2004 (2004-12-24), pages 54264-54274, XP002339040 cited in the application

### SEQUENCE LISTING

<110> CropDesign N.V.
<120> Plants having modified growth characteristics and method for making the same
<130> CD-107-PCT
<150> EP 03104764.0
   <151> 2003-12-17
<150> US 60/531,866
   <151> 2003-12-22
<160> 7
<170> PatentIn version 3.3
<210> 1
   <211> 1380
   <212> DNA
   <213> Nicotiana tabacum
<400> 1
   atgggtgaca tgaaggataa agtcaaaggg ttcatgaaaa aagtcacatc ttcttcttca     60
   ggtaagttta aaggccaagg tagggttttg ggtggttcat cttcttcagg accctcaaat    120
   catgtcaata atttttcatc acatccccta aatacaaggc aagatcaaca accttcatat    180
   acaaaaactt cgcctcaaaa accaagtaat tctgatcaaa gaattgagaa tatatgtgaa    240
   attcagttca acaaaagtga atcaaaggat ggttttgatc catttggtga attagtcact    300
   tctgggaaga gaaacccaaa agggtattca cttactaatg tgtttgaatg ccctgtctgt    360
   ggtagtggtt ttgtttctga agaagaggtg tcaactcata ttgatagctg tttaagttct    420
   gaagtgtctt ctaatttggg agttgaaagt aaagttgaag ttaaaagtga attggaaaca    480
   tgtgttagtg catatgtttc agggaagccc tcagaagggt cagttgaagt ggtcattaag    540
   ttgttaaaga atattgtgaa ggaaccagag aatgccaagt ttaggaaaat aaggatgggg    600
   aatccaaaaa taaaaggtgc tataggtgat gttgtaggag gagtggagct attggaattt    660
   gttggatttg agttgaaaga agaaggtggg gaaatttggg ctgtgatgga tgttccttct    720
   gaagaacaac ttgttatgct taagaatgta gtttcactct tggaaccgaa gaaggttgaa    780
   gagttggcgt ccttatccca agttaaggcg agtgaaccag ttgagccgaa gaagattgat    840
   agacagattc gagtgttctt ttctgttccc gagagcgtag cagcaaaaat tgagctacct    900
   gattccttct ttaacctctc acgtgaggaa ttgagaagag aagcagagat gaggaagaag    960
   aaattagaag attccaaatt attgattcct aaatcttatc gggaaaagca ggcaaaagct   1020
   gcaagaaaga agtacacaaa atccattatc cgtgtacagt ttccagatgg agcattgctt   1080
   caaggtgtct ttctaccttc ggagccaact agtgctcttt atgagtttgt gagcgcagcg   1140
   ttaaaggaac caagcttaga gttcgaattg ttacatccgg tgcttgttaa aaagcgggtg   1200
   attccccatt ttccagctgc tggggagagg gctgtaacag ttgaagagga ggatttggtt   1260
   cctgcagctc tactcaaatt taaacctatc gaaacagatt ctgttgtttt tactggtctt   1320
   tgtaatgagc ttcttgaaat tagcgagccc ctcgagaccg gatcagttgc ttcctcgtaa   1380
<210> 2
   <211> 459
   <212> PRT
   <213> Nicotiana tabacum
<400> 2
   Met Gly Asp Met Lys Asp Lys Val Lys Gly Phe Met Lys Lys Val Thr
   1               5                   10                  15
   Ser Ser Ser Ser Gly Lys Phe Lys Gly Gln Gly Arg Val Leu Gly Gly
               20                  25                  30
   Ser Ser Ser Ser Gly Pro Ser Asn His Val Asn Asn Phe Ser Ser His
           35                  40                  45
   Pro Leu Asn Thr Arg Gln Asp Gln Gln Pro Ser Tyr Thr Lys Thr Ser
       50                  55                  60
   Pro Gln Lys Pro Ser Asn Ser Asp Gln Arg Ile Glu Asn Ile Cys Glu
   65                  70                  75                  80
   Ile Gln Phe Asn Lys Ser Glu Ser Lys Asp Gly Phe Asp Pro Phe Gly
                   85                  90                  95
   Glu Leu Val Thr Ser Gly Lys Arg Asn Pro Lys Gly Tyr Ser Leu Thr
               100                 105                 110
   Asn Val Phe Glu Cys Pro Val Cys Gly Ser Gly Phe Val Ser Glu Glu
           115                 120                 125
   Glu Val Ser Thr His Ile Asp Ser Cys Leu Ser Ser Glu Val Ser Ser
       130                 135                 140
   Asn Leu Gly Val Glu Ser Lys Val Glu Val Lys Ser Glu Leu Glu Thr
   145                 150                 155                 160
   Cys Val Ser Ala Tyr Val Ser Gly Lys Pro Ser Glu Gly Ser Val Glu
                   165                 170                 175
   Val Val Ile Lys Leu Leu Lys Asn Ile Val Lys Glu Pro Glu Asn Ala
               180                 185                 190
   Lys Phe Arg Lys Ile Arg Met Gly Asn Pro Lys Ile Lys Gly Ala Ile
           195                 200                 205
   Gly Asp Val Val Gly Gly Val Glu Leu Leu Glu Phe Val Gly Phe Glu
       210                 215                 220
   Leu Lys Glu Glu Gly Gly Glu Ile Trp Ala Val Met Asp Val Pro Ser
   225                 230                 235                 240
   Glu Glu Gln Leu Val Met Leu Lys Asn Val Val Ser Leu Leu Glu Pro
                   245                 250                 255
   Lys Lys Val Glu Glu Leu Ala Ser Leu Ser Gln Val Lys Ala Ser Glu
               260                 265                 270
   Pro Val Glu Pro Lys Lys Ile Asp Arg Gln Ile Arg Val Phe Phe Ser
           275                 280                 285
   Val Pro Glu Ser Val Ala Ala Lys Ile Glu Leu Pro Asp Ser Phe Phe
       290                 295                 300
   Asn Leu Ser Arg Glu Glu Leu Arg Arg Glu Ala Glu Met Arg Lys Lys
   305                 310                 315                 320
   Lys Leu Glu Asp Ser Lys Leu Leu Ile Pro Lys Ser Tyr Arg Glu Lys
                   325                 330                 335
   Gln Ala Lys Ala Ala Arg Lys Lys Tyr Thr Lys Ser Ile Ile Arg Val
               340                 345                 350
   Gln Phe Pro Asp Gly Ala Leu Leu Gln Gly Val Phe Leu Pro Ser Glu
           355                 360                 365
   Pro Thr Ser Ala Leu Tyr Glu Phe Val Ser Ala Ala Leu Lys Glu Pro
       370                 375                 380
   Ser Leu Glu Phe Glu Leu Leu His Pro Val Leu Val Lys Lys Arg Val
   385                 390                 395                 400
   Ile Pro His Phe Pro Ala Ala Gly Glu Arg Ala Val Thr Val Glu Glu
                   405                 410                 415
   Glu Asp Leu Val Pro Ala Ala Leu Leu Lys Phe Lys Pro Ile Glu Thr
               420                 425                 430
   Asp Ser Val Val Phe Thr Gly Leu Cys Asn Glu Leu Leu Glu Ile Ser
           435                 440                 445
   Glu Pro Leu Glu Thr Gly Ser Val Ala Ser Ser
       450                 455
<210> 3
   <211> 1311
   <212> DNA
   <213> Saccharum officinarum
<220>
   <221> misc_feature
   <222> (277)..(279)
   <223> n can be any nucleotide
<400> 3
   atgatgaagg acaagatgaa ggagttcatg aagaaggtca cctcctccgg gtccgggacc      60
   ccctcctcct tcaagggcac ctcccacgtc ctcggctccg gcccctcccc ctcctcctcc     120
   caccccgctg cccgctcctc aaaccctagc ccaaacctca ggcccgctcc taagcggacc     180
   tcgccaccta ccccgcccac tttaaccacc gatttgacct ccttcacgcc cctcgtctgc     240
   tactcctccc gccgccccga cgcgaacggc accgcgnnng ccgtcgccac cgtcgcgtgc     300
   cccagctgcg gagacgcgtt tccgtccgag ctcgccgtct ccgagcatct cgacggctgc     360
   ctcgcgtcgg cggggggcgc ccgcgcgcgc gccgccgcgt acctcgccgc cgacccgcct      420
   ccgcccgcgg cctccgtaga ggtggtcaaa cgcctgctgg gcaacctgct ccgggagccc     480
   ggcaacgata agttcaggcg ggtgagattg ggtaacccgc ggatcaagga ggccctggca     540
   gacagggatg gcggggtgga gctcctggag gccgtcggct tcacagttgg ggatgagggc     600
   ggggagccct tcgccgtgat ggacgaagtg cctagcgacc ctaggctcaa cgggatcagg     660
   agggccgtcc tcctgctcga gggggcacac ccctctgcgc ctccagtgaa ggcggaggct     720
   gaggccaagg agagctgcag caatgtgtct gacgtgcagg agggtgctaa gactattgat     780
   cggcagattc gggtatttgt ctctgttcct gggagttcta tggcacaaaa tgatgtacca     840
   gattcttttt acaagcttag tggtgaggag ataaggaatg aagcaaagat gaggagggaa     900
   aggttagaac aatctcgatt gctgatacca aagtcttaca aggagaaaca ggcattggct     960
   gctcgacaga agtataaaca agcagtcatt cgagttcagt ttccagatag aatgattctt    1020
   cagggcatat tcctaccagg agaggccact agttcactgt atgagttcgt cacatctgct    1080
   ctgaagcaat caggtttgga attcgaactt atctctccag ccatacctaa gccacgtgtg    1140
   gtgccccatt ttccaaaccc gggagagcgg gcacgcacct tgcaagagga ggagctggtc    1200
   ccatctgcgc tcctcaagtt cattcccaag gagactgatt ccatggtttt caccggtttg    1260
   cttgatgagc ttctcatggc cagtgagccg cttcctgctg catcacaatg a             1311
<210> 4
   <211> 436
   <212> PRT
   <213> Saccharum officinarum
<220>
   <221> MISC_FEATURE
   <222> (93)..(93)
   <223> Xaa can be any amino acid
<400> 4
   Met Met Lys Asp Lys Met Lys Glu Phe Met Lys Lys Val Thr Ser Ser
   1               5                   10                  15
   Gly Ser Gly Thr Pro Ser Ser Phe Lys Gly Thr Ser His Val Leu Gly
               20                  25                  30
   Ser Gly Pro Ser Pro Ser Ser Ser His Pro Ala Ala Arg Ser Ser Asn
           35                  40                  45
   Pro Ser Pro Asn Leu Arg Pro Ala Pro Lys Arg Thr Ser Pro Pro Thr
       50                  55                  60
   Pro Pro Thr Leu Thr Thr Asp Leu Thr Ser Phe Thr Pro Leu Val Cys
   65                  70                  75                  80
   Tyr Ser Ser Arg Arg Pro Asp Ala Asn Gly Thr Ala Xaa Ala Val Ala
                   85                  90                  95
   Thr Val Ala Cys Pro Ser Cys Gly Asp Ala Phe Pro Ser Glu Leu Ala
               100                 105                 110
   Val Ser Glu His Leu Asp Gly Cys Leu Ala Ser Ala Gly Gly Ala Arg
           115                 120                 125
   Ala Arg Ala Ala Ala Tyr Leu Ala Ala Asp Pro Pro Pro Pro Ala Ala
       130                 135                 140
   Ser Val Glu Val Val Lys Arg Leu Leu Gly Asn Leu Leu Arg Glu Pro
   145                 150                 155                 160
   Gly Asn Asp Lys Phe Arg Arg Val Arg Leu Gly Asn Pro Arg Ile Lys
                   165                 170                 175
   Glu Ala Leu Ala Asp Arg Asp Gly Gly Val Glu Leu Leu Glu Ala Val
               180                 185                 190
   Gly Phe Thr Val Gly Asp Glu Gly Gly Glu Pro Phe Ala Val Met Asp
           195                 200                 205
   Glu Val Pro Ser Asp Pro Arg Leu Asn Gly Ile Arg Arg Ala Val Leu
       210                 215                 220
   Leu Leu Glu Gly Ala His Pro Ser Ala Pro Pro Val Lys Ala Glu Ala
   225                 230                 235                 240
   Glu Ala Lys Glu Ser Cys Ser Asn Val Ser Asp Val Gln Glu Gly Ala
                   245                 250                 255
   Lys Thr Ile Asp Arg Gln Ile Arg Val Phe Val Ser Val Pro Gly Ser
                   260                 265                 270
   Ser Met Ala Gln Asn Asp Val Pro Asp Ser Phe Tyr Lys Leu Ser Gly
           275                 280                 285
   Glu Glu Ile Arg Asn Glu Ala Lys Met Arg Arg Glu Arg Leu Glu Gln
       290                 295                 300
   Ser Arg Leu Leu Ile Pro Lys Ser Tyr Lys Glu Lys Gln Ala Leu Ala
   305                 310                 315                 320
   Ala Arg Gln Lys Tyr Lys Gln Ala Val Ile Arg Val Gln Phe Pro Asp
                   325                 330                 335
   Arg Met Ile Leu Gln Gly Ile Phe Leu Pro Gly Glu Ala Thr Ser Ser
               340                 345                 350
   Leu Tyr Glu Phe Val Thr Ser Ala Leu Lys Gln Ser Gly Leu Glu Phe
           355                 360                 365
   Glu Leu Ile Ser Pro Ala Ile Pro Lys Pro Arg Val Val Pro His Phe
       370                 375                 380
   Pro Asn Pro Gly Glu Arg Ala Arg Thr Leu Gln Glu Glu Glu Leu Val
   385                 390                 395                 400
   Pro Ser Ala Leu Leu Lys Phe Ile Pro Lys Glu Thr Asp Ser Met Val
                   405                 410                 415
   Phe Thr Gly Leu Leu Asp Glu Leu Leu Met Ala Ser Glu Pro Leu Pro
               420                 425                 430
   Ala Ala Ser Gln
           435
<210> 5
   <211> 3048
   <212> DNA
   <213> Artificial sequence
<220>
   <223> expression cassette comprising GRUBX (1011-2390) operably linked to the prolamine promoter (1-654) and the T-Zein + T-Rubisco deltaG terminator (2615-2808 and 2852-3048)
<400> 5
   cttctacatc ggcttaggtg tagcaacacg actttattat tattattatt attattatta     60
   ttattttaca aaaatataaa atagatcagt ccctcaccac aagtagagca agttggtgag    120
   ttattgtaaa gttctacaaa gctaatttaa aagttattgc attaacttat ttcatattac    180
   aaacaagagt gtcaatggaa caatgaaaac catatgacat actataattt tgtttttatt    240
   attgaaatta tataattcaa agagaataaa tccacatagc cgtaaagttc tacatgtggt    300
   gcattaccaa aatatatata gcttacaaaa catgacaagc ttagtttgaa aaattgcaat    360
   ccttatcaca ttgacacata aagtgagtga tgagtcataa tattattttc tttgctaccc    420
   atcatgtata tatgatagcc acaaagttac tttgatgatg atatcaaaga acatttttag    480
   gtgcacctaa cagaatatcc aaataatatg actcacttag atcataatag agcatcaagt    540
   aaaactaaca ctctaaagca accgatggga aagcatctat aaatagacaa gcacaatgaa    600
   aatcctcatc atccttcacc acaattcaaa tattatagtt gaagcatagt agtaatttaa    660
   atcaactagg gatatcacaa gtttgtacaa aaaagcaggc tggtaccggt ccggaattcc    720
   cgggatatcg tcgacccacg cgtccgccaa tatcagattt ctttcatgaa ctccacttcc    780
   aatttctcat tgcttcttct tcccatttcc acctccaaag ccatccttcc agaaaacctt     840
   gttccttaca tttcttagcc ccaaaaaaga ttcccatctc aattccacaa aaaaacacaa     900
   ggagatctaa ggaaattccc cgcctctata tatagagagg tggaattgtt cctgaatttg     960
   gtttgaattg attgattgac agattttggt gagagggtgt tattgaaaaa atgggtgaca    1020
   tgaaggataa agtcaaaggg ttcatgaaaa aagtcacatc ttcttcttca ggtaagttta    1080
   aaggccaagg tagggttttg ggtggttcat cttcttcagg accctcaaat catgtcaata    1140
   atttttcatc acatccccta aatacaaggc aagatcaaca accttcatat acaaaaactt    1200
   cgcctcaaaa accaagtaat tctgatcaaa gaattgagaa tatatgtgaa attcagttca    1260
   acaaaagtga atcaaaggat ggttttgatc catttggtga attagtcact tctgggaaga    1320
   gaaacccaaa agggtattca cttactaatg tgtttgaatg ccctgtctgt ggtagtggtt    1380
   ttgtttctga agaagaggtg tcaactcata ttgatagctg tttaagttct gaagtgtctt    1440
   ctaatttggg agttgaaagt aaagttgaag ttaaaagtga attggaaaca tgtgttagtg    1500
   catatgtttc agggaagccc tcagaagggt cagttgaagt ggtcattaag ttgttaaaga    1560
   atattgtgaa ggaaccagag aatgccaagt ttaggaaaat aaggatgggg aatccaaaaa    1620
   taaaaggtgc tataggtgat gttgtaggag gagtggagct attggaattt gttggatttg    1680
   agttgaaaga agaaggtggg gaaatttggg ctgtgatgga tgttccttct gaagaacaac    1740
   ttgttatgct taagaatgta gtttcactct tggaaccgaa gaaggttgaa gagttggcgt    1800
   ccttatccca agttaaggcg agtgaaccag ttgagccgaa gaagattgat agacagattc    1860
   gagtgttctt ttctgttccc gagagcgtag cagcaaaaat tgagctacct gattccttct    1920
   ttaacctctc acgtgaggaa ttgagaagag aagcagagat gaggaagaag aaattagaag    1980
   attccaaatt attgattcct aaatcttatc gggaaaagca ggcaaaagct gcaagaaaga    2040
   agtacacaaa atccattatc cgtgtacagt ttccagatgg agcattgctt caaggtgtct    2100
   ttctaccttc ggagccaact agtgctcttt atgagtttgt gagcgcagcg ttaaaggaac    2160
   caagcttaga gttcgaattg ttacatccgg tgcttgttaa aaagcgggtg attccccatt    2220
   ttccagctgc tggggagagg gctgtaacag ttgaagagga ggatttggtt cctgcagctc    2280
   tactcaaatt taaacctatc gaaacagatt ctgttgtttt tactggtctt tgtaatgagc    2340
   ttcttgaaat tagcgagccc ctcgagaccg gatcagttgc ttcctcgtaa gctctaaatt    2400
   acatcagact ttgaattctt ctgagtgttg gaaaccttat aaaactctct gcgccgggaa    2460
   tgctgcggcc gctctagagt atccctcgag gggcccaagc ttacgcgtac ccagctttct    2520
   tgtacaaagt ggtgatatca caagcccggg cggtcttcta gggataacag ggtaattata    2580
   tccctctaga tcacaagccc gggcggtctt ctacgatgat tgagtaataa tgtgtcacgc    2640
   atcaccatgg gtggcagtgt cagtgtgagc aatgacctga atgaacaatt gaaatgaaaa    2700
   gaaaaaaagt actccatctg ttccaaatta aaattcattt taacctttta ataggtttat    2760
   acaataattg atatatgttt tctgtatatg tctaatttgt tatcatccgg gcggtcttct    2820
   agggataaca gggtaattat atccctctag acaacacaca acaaataaga gaaaaaacaa    2880
   ataatattaa tttgagaatg aacaaaagga ccatatcatt cattaactct tctccatcca    2940
   tttccatttc acagttcgat agcgaaaacc gaataaaaaa cacagtaaat tacaagcaca    3000
   acaaatggta caagaaaaac agttttccca atgccataat actcgaac                 3048
<210> 6
   <211> 1302
   <212> DNA
   <213> Oryza sativa
<400> 6
   atgatgaagg aaaagatgaa ggatctcatg aggaaggtca cctcctcctc ctcctcctcc     60
   tcgtcgtcct cctccttcaa gggcaccgcc cacgtcctcg gctccggccc cgacccctcc    120
   tcccgcccct ccaaccctac ccctagtcgc cccgctgccc cccggcgaga ggccgccgcc    180
   tccgcgaggc cgccctcctc cggcttcgcc ccctactccc cgctcatctc cacctcctcc    240
   cgccgcaccg acccacccgc gggggcgggg gcgggggagg acgacgccgt cgcgtgcccc    300
   agctgcgccg agccgttccc ctccgagctg gcggtgtcgg accacctcga cggctgcctc    360
   gcggcggcgg ggggagcccg cccccgcgcg gccgcctacc tggccggcga cccccccgcg    420
   tccgccgtgg aggtggtgaa gaggctgctc gggaacctgc tctccgaccc ccggaacgac    480
   aagtacagga aggtcaggct cgggaacccg aggatcaagg aggccctggc ggacagggag    540
   ggcggggtgg atctcctcga ggccgtgggg ttcagggtcg ccgacgaggg cggggagctc    600
   ttcgccctca tggacgaggt gcccggggac gcgaggctcg gcggcatcag gcaggccgtg    660
   ctcctgctcg agagggcccg gccatcgacg ccgccgcaga cacaggcaga tgccaaagag    720
   acttgcccga atggagttag cgaagagcag gggattaaga agccggttga tcgtcagatt    780
   cgggtgttct tctctgttgc tgcaagttct gttgcagaaa atgatctacc agattctttc    840
   tatagcctta gtaatgagga gatcaggaat gaggcaaaga tgaggaggga gaggctagaa    900
   caatctcggt tgttgattcc aaagtcatat aaggagaagc aggcactggc tgcccgacag    960
   aagtataaac aagctctgat tcgaattcag tttccggatg gagtaattct gcagggtgtg   1020
   ttccttcccg cggagcccat tagttcacta tatgagtttg tcgcatcttc tttgaagcag   1080
   ccaagtttgg aattcgatct tatctgtcca gcgggtccta ggacacgcgt catacctcct   1140
   tttccaaaac caggggaaca ggcacgcaca ctgcgggatg aagacctagt cccttctgct   1200
   cggctcacct tcaagcccaa agagactgat tcggttgtgt tcactggtct gttggacgag   1260
   cttcttgaga ccagtgagcc gttcacatct gcatcctcat ga                       1302
<210> 7
   <211> 433
   <212> PRT
   <213> Oryza sativa
<400> 7
   Met Met Lys Glu Lys Met Lys Asp Leu Met Arg Lys Val Thr Ser Ser
   1               5                   10                  15
   Ser Ser Ser Ser Ser Ser Ser Ser Ser Phe Lys Gly Thr Ala His Val
               20                  25                  30
   Leu Gly Ser Gly Pro Asp Pro Ser Ser Arg Pro Ser Asn Pro Thr Pro
           35                  40                  45
   Ser Arg Pro Ala Ala Pro Arg Arg Glu Ala Ala Ala Ser Ala Arg Pro
       50                  55                  60
   Pro Ser Ser Gly Phe Ala Pro Tyr Ser Pro Leu Ile Ser Thr Ser Ser
   65                  70                  75                  80
   Arg Arg Thr Asp Pro Pro Ala Gly Ala Gly Ala Gly Glu Asp Asp Ala
                   85                  90                  95
   Val Ala Cys Pro Ser Cys Ala Glu Pro Phe Pro Ser Glu Leu Ala Val
               100                 105                 110
   Ser Asp His Leu Asp Gly Cys Leu Ala Ala Ala Gly Gly Ala Arg Pro
           115                 120                 125
   Arg Ala Ala Ala Tyr Leu Ala Gly Asp Pro Pro Ala Ser Ala Val Glu
       130                 135                 140
   Val Val Lys Arg Leu Leu Gly Asn Leu Leu Ser Asp Pro Arg Asn Asp
   145                 150                 155                 160
   Lys Tyr Arg Lys Val Arg Leu Gly Asn Pro Arg Ile Lys Glu Ala Leu
                   165                 170                 175
   Ala Asp Arg Glu Gly Gly Val Asp Leu Leu Glu Ala Val Gly Phe Arg
               180                 185                 190
   Val Ala Asp Glu Gly Gly Glu Leu Phe Ala Leu Met Asp Glu Val Pro
           195                 200                 205
   Gly Asp Ala Arg Leu Gly Gly Ile Arg Gln Ala Val Leu Leu Leu Glu
       210                 215                 220
   Arg Ala Arg Pro Ser Thr Pro Pro Gln Thr Gln Ala Asp Ala Lys Glu
   225                 230                 235                 240
   Thr Cys Pro Asn Gly Val Ser Glu Glu Gln Gly Ile Lys Lys Pro Val
                   245                 250                 255
   Asp Arg Gln Ile Arg Val Phe Phe Ser Val Ala Ala Ser Ser Val Ala
               260                 265                 270
   Glu Asn Asp Leu Pro Asp Ser Phe Tyr Ser Leu Ser Asn Glu Glu Ile
           275                 280                 285
   Arg Asn Glu Ala Lys Met Arg Arg Glu Arg Leu Glu Gln Ser Arg Leu
       290                 295                 300
   Leu Ile Pro Lys Ser Tyr Lys Glu Lys Gln Ala Leu Ala Ala Arg Gln
   305                 310                 315                 320
   Lys Tyr Lys Gln Ala Leu Ile Arg Ile Gln Phe Pro Asp Gly Val Ile
                   325                 330                 335
   Leu Gln Gly Val Phe Leu Pro Ala Glu Pro Ile Ser Ser Leu Tyr Glu
               340                 345                 350
   Phe Val Ala Ser Ser Leu Lys Gln Pro Ser Leu Glu Phe Asp Leu Ile
           355                 360                 365
   Cys Pro Ala Gly Pro Arg Thr Arg Val Ile Pro Pro Phe Pro Lys Pro
       370                 375                 380
   Gly Glu Gln Ala Arg Thr Leu Arg Asp Glu Asp Leu Val Pro Ser Ala
   385                 390                 395                 400
   Arg Leu Thr Phe Lys Pro Lys Glu Thr Asp Ser Val Val Phe Thr Gly
                   405                 410                 415
   Leu Leu Asp Glu Leu Leu Glu Thr Ser Glu Pro Phe Thr Ser Ala Ser
               420                 425                 430
   Ser

## Description

The present invention concerns a method for improving plant growth characteristics. More specifically, the present invention concerns a method for improving seed yield by introducing and overexpressing a nucleic acid encoding a GRUBX in a plant or plant cell and cultivating the plant or plant cell under conditions promoting plant growth..

Given the ever-increasing world population, and the dwindling area of land available for agriculture, it remains a major goal of agricultural research to improve the efficiency of agriculture and to increase the diversity of plants in horticulture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Furthermore, suitable donor species for providing a desired trait may be scarce. Advances in molecular biology have allowed mankind to manipulate the germplasm of animals and plants. Genetic engineering of plants entails the isolation and manipulation of genetic material (typically in the form of DNA or RNA) and the subsequent introduction of that genetic material into a plant. Such technology has led to the development of plants having various improved economic, agronomic or horticultural traits. Traits of particular economic interest are growth characteristics such as high yield. Yield is normally defined as the measurable produce of economic value from a crop. This may be defined in terms of quantity and/or quality. Crop yield is adversely influenced by the typical stresses to which plants or crops are subjected. Such stresses include abiotic stresses, such as temperature stresses caused by atypical high or low temperatures; stresses caused by nutrient deficiency; stresses caused by a lack of or excess water (drought, flooding), stresses caused by chemicals such as fertilisers or insecticides. Typical stresses also include biotic stresses, which may be imposed on plants by other plants (weeds, or the effects of high density planting), by animal pests (including stresses caused by grazing), and by pathogens. Crop yield may not only be increased by combating one or more of the stresses to which the crop or plant is subjected, but may also be increased by modifying the inherent growth mechanisms of a plant. The inherent growth mechanisms of a plant are controlled at several levels and by various metabolic processes. One such process is the control of protein levels in a cell by ubiquitin-mediated protein degradation.

Ubiquitination refers to a modification of proteins by conjugation to ubiquitin molecules. The term ubiquitination is often extended to processes that mediate binding of ubiquitin proteins or of proteins that mimic ubiquitin function. Ubiquitination is a versatile tool for eukaryotic cells to control stability, function and the subcelullar localisation of proteins. This mechanism plays a central role in protein degradation, cell cycle control, stress responses, DNA repair, signal transduction, transcriptional regulation and vesicular trafficking. Since ubiquitin mediated protein degradation is at the basis of many cellular processes, it is highly regulated and requires high substrate specificity and ample diversity in downstream effectors. Several ubiquitin-binding proteins are known. These proteins have often a modular domain architecture. For example, ubiquitin-binding proteins typically combine a ubiquitin binding domain with a variable effector domain. Then there are others that do not contain a ubiquitin binding domain, but have a tertiary structure similar to ubiquitin and can therefore mimic certain aspects of ubiquitination (ubiquitin-like domains).

The number of ubiquitin-related motifs and domains present in ubiquitin and ubiquitin-like proteins is growing as more information on genome sequences becomes available. Some prototypes of those domains are for example UBA, UBD, UIM and UBX (see for example the Pfam database; Bateman et al., Nucleic Acids Research 30(1):276-280 (2002)). The UBX domain is a sequence approximately 80 amino acid residues long, is of unknown function and is present in proteins of various organisms. Most of these proteins belong to one of five evolutionary conserved families exemplified by the human FAF1, p47, Y33K, REP8, and UBXD1 proteins (Buchberger et al. (2001) J. Mol. Biol. 307, 17-24; Carim-Todd et al. (2001) Biochim. Biophys. Acta 1517, 298-301). Typically, the UBX domain is situated at the C-terminus of a protein.

Structural evidence suggests a function of the UBX domain in ubiquitin-related processes; in particular the UBX domain may be involved in protein-protein interactions. Proteins comprising UBX domains are usually predicted to be present mainly in the cytoplasm, but other subcellular localizations have also been reported. For example, phosphorylation which is a specific protein modification used to regulate activity of many proteins, has been shown to also influence transport into the nucleus of FAF-1 (Olsen et al. (2003) FEBS Lett. 546, 218-222.). In summary, it has been proposed that animal UBX-containing proteins might be involved in enhanced expression of genes related to apoptosis, cell cycling or targeting of proteins for degradation.

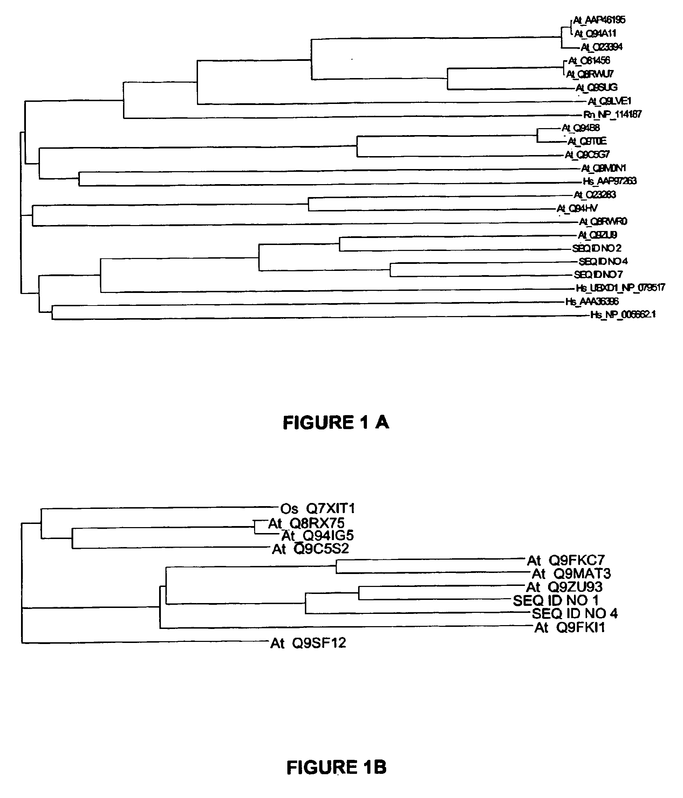

In *Arabidopsis,* the genome of which plant has been fully sequenced, there are at least 15 UBX-containing proteins. They may be classified according to sequence similarity in the FAF1, p47, Y33K and UBXD1 groups, only the group corresponding to REP8 appears not to be present in plants (see Figure 1). As in the animal kingdom, the UBX domains in plant proteins are present in combination with other domains, like for example SEP, G6PD, PUG, or zinc fingers. UBX-containing proteins and the domain structure of these proteins have been described (see Buchberger (2002) Trends Cell Biol. 12, 216-221) and can be identified by searching using specialised databases such as SMART (Schultz et al. (1998) Proc. Natl. Acad. Sci. USA 95, 5857-5864; Letunic et al. (2002) Nucleic Acids Res 30, 242-244).

PUG domains (in Peptide:N-Glycanases and other putative nuclear UBX-domain-containing proteins; Doerks et al. (2002) Genome Research 12, 47-56) co-occur in proteins with domains that are central to ubiquitin-mediated proteolysis, including UBX (in mammals and plants), UBA (in plants) and UBC domains (in *Plasmodium).* PUG-containing proteins such as PNGases are believed to play a role in the unfolded protein response, an endoplasmatic reticulum (ER) quality control surveillance system that distinguishes aberrant proteins from correctly folded proteins. In some cases, it has been shown that these misfolded and/or unfolded proteins are degraded by a so-called ER-associated degradation mechanism, which involves the ubiquitin-proteasome system (Suzuki et al. (2000) J. Cell Biol. 149, 1039-1052). Divergent forms of PUG domains are also present in kinases of the IRE1p type which are known to function in the initial stages of the unfolded protein response (Shamu and Walter (1996) EMBO J. 15, 3028-3039).

A recently characterised *Arabidopsis* protein comprising an UBX domain is PUX1 (Rancour et al. (2004) J. Biol. Chem., online publication 10.1074/jbc.M405498200). *PUX1* is a single gene in *Arabidopsis* and is probably expressed ubiquitously *in planta.* The protein was shown to be a non-competitive inhibitor of the AAA-type ATPase CDC48. PUX1 associates through its UBX domain with the non-hexameric form of CDC48, but not with the hexameric CDC48. It is postulated that PUX1 facilitates the disassembly of active hexameric CDC48 and that the N-terminal domain of the protein is required for this process. *pux1* knockout plants showed a faster development to maturity but had no gross morphological abnormalities. Besides PUX1, two other UBX domain comprising proteins, PUX2 and PUX3, were shown to interact with CDC48 (Rancour et al., 2004). PUX2 (At2g01650) was previously disclosed in WO 03/085115 (gene and protein sequence described as SEQ ID NO: 1 and SEQ ID NO: 2 respectively).

It has now been found that introducing and overexpressing a nucleic acid encoding a GRUBX protein (Growth Related UBX domain-comprising protein), and in particular a nucleic acid encoding the GRUBX protein exemplified by SEQ ID NO: 2, in a plant gives plants having improved harvest index relative to corresponding wild type plants. Therefore, according to a first embodiment of the present invention there is provided a method for improving seed yield of a plant, comprising introducing and overexpressing in a plant a nucleic acid sequence encoding a GRUBX protein, said nucleic acid sequence being selected from the group:
- a nucleic acid sequence as represented by any one of SEQ ID NO:1, 3, 6;
- a nucleic acid encoding a polypeptide as represented by any one of SEQ ID NO: 2, 4, 7;
- a nucleic acid encoding a polypeptide having at least 80% sequence identity to any one of SEQ ID NO: 2, 4, 7, said polypeptide having ubiquitin-regulatory action within the ubiquitination pathway and cultivating the plant or plant cell under conditions promoting plant growth.. Optionally, plants having improved seed yield may be selected for.

Homologous recombination allows introduction in a genome of a selected nucleic acid at a defined selected position. Homologous recombination is a standard technology used routinely in biological sciences for lower organism such as yeast and the moss *Physcomitrella.* Methods for performing homologous recombination in plants have been described not only for model plants (Offringa et al. (1990) EMBO J. 9, 3077-3084) but also for crop plants, for example rice (Terada et al., (2002) Nature Biotechnol. 20, 1030-1034; or lida and Terada (2004) Curr. Opin. Biotechnol. 15, 132-138). The nucleic acid to be targeted (which may be a *GRUBX* nucleic acid molecule or variant thereof as hereinbefore defined) need not be targeted to the locus of a *GRUBX* gene, but may be introduced in, for example, regions of high expression. The nucleic acid to be targeted may be an improved allele used to replace the endogenous gene or may be introduced in addition to the endogenous gene.

The nucleic acid may be introduced into a plant by, for example, transformation.

The term GRUBX protein, as defined herein, refers to a nucleic acid encoding a polypeptide having at least 80% sequence identity to any one of SEQ ID NO: 2, 4, 7, said polypeptide having ubiquitin-regulatory action within the ubiquitination pathway , and optionally also a Zinc finger domain. Preferably, the GRUBX protein is structurally related to the human UBXD1 protein (SPTrEMBL AAH07414). Preferably, the GRUBX protein is from a plant. Further preferably, the GRUBX protein is from the family of Solanaceae, more preferably the GRUBX is a protein from *Nicotiana tabacum,* most preferably the GRUBX is a protein as represented by SEQ ID NO: 2 or a homologue, derivative or active fragment thereof, which homologues, derivatives or active fragments have similar biological activity to that of SEQ ID NO: 2. However, it should be understood that GRUBX proteins from monocotyledonous plants could equally well be used in the methods of the present invention, including GRUBX proteins from *Zea mays, Saccharum officinarum* (SEQ ID NO 4), *Oryza sativa* (SEQ ID NO 7), *Triticum* sp., *Hordeum* sp., and *Sorghum* sp, since these sequences are related to SEQ ID NO 2 (see Figure 1b).

One of the activities of a GRUBX protein is increasing seed yield, in particular increasing harvest index, when a nucleic acid encoding such GRUBX protein is expressed in rice under control of a prolamin promoter as used in the present invention. Advantageously, a GRUBX protein is able to interact with plant CDC48 proteins under conditions described in Rancour et al. (2004).

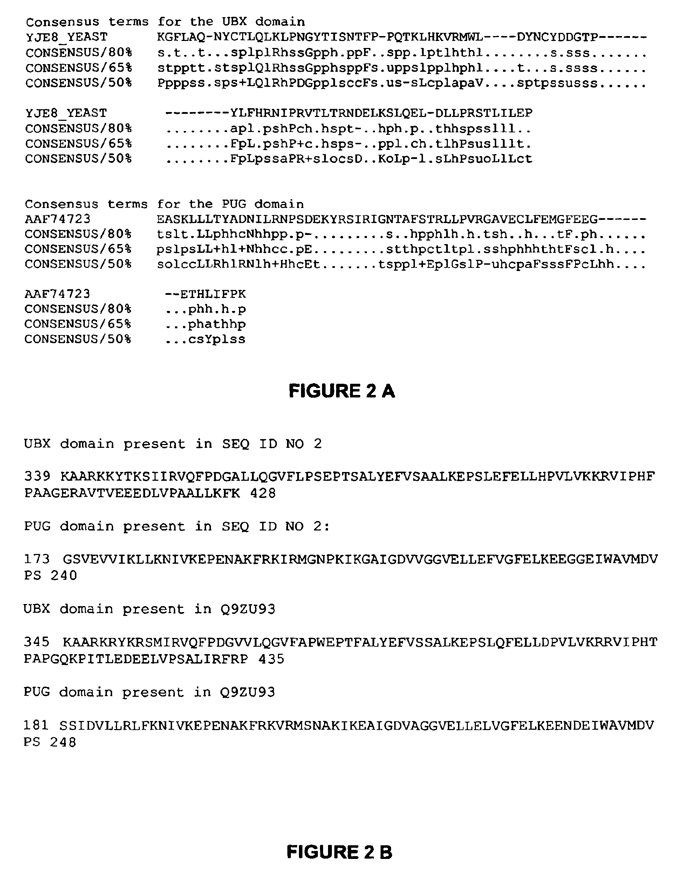

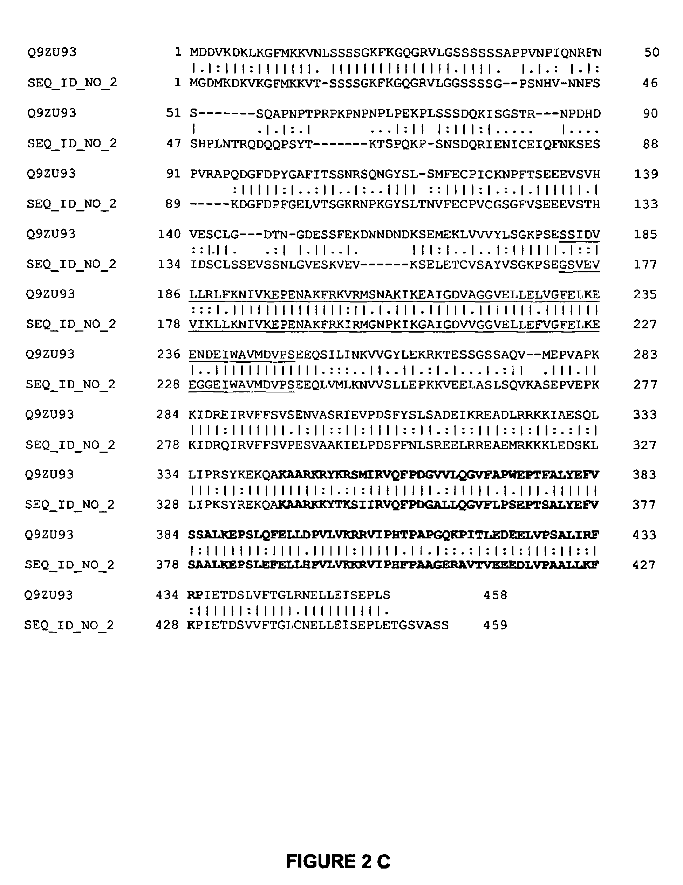

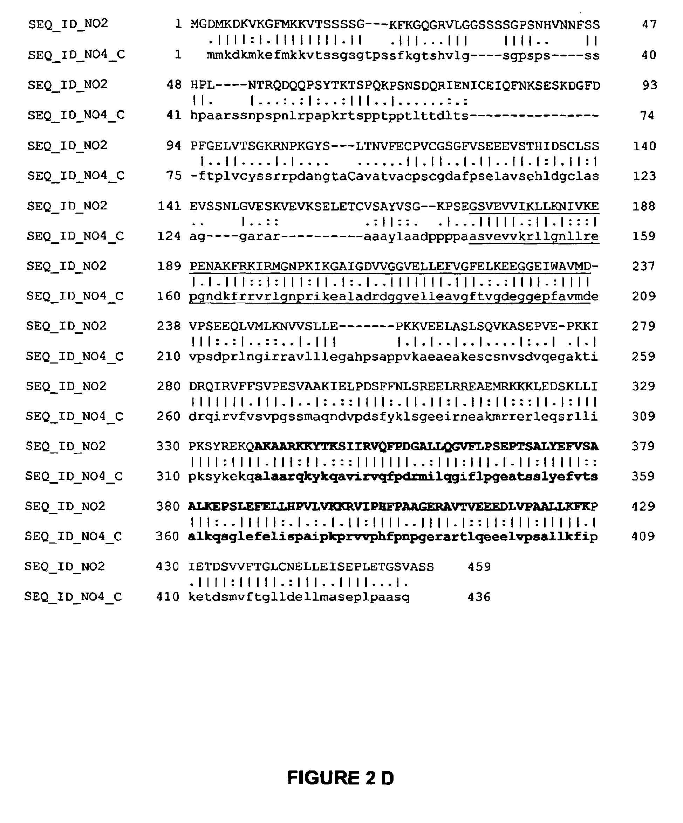

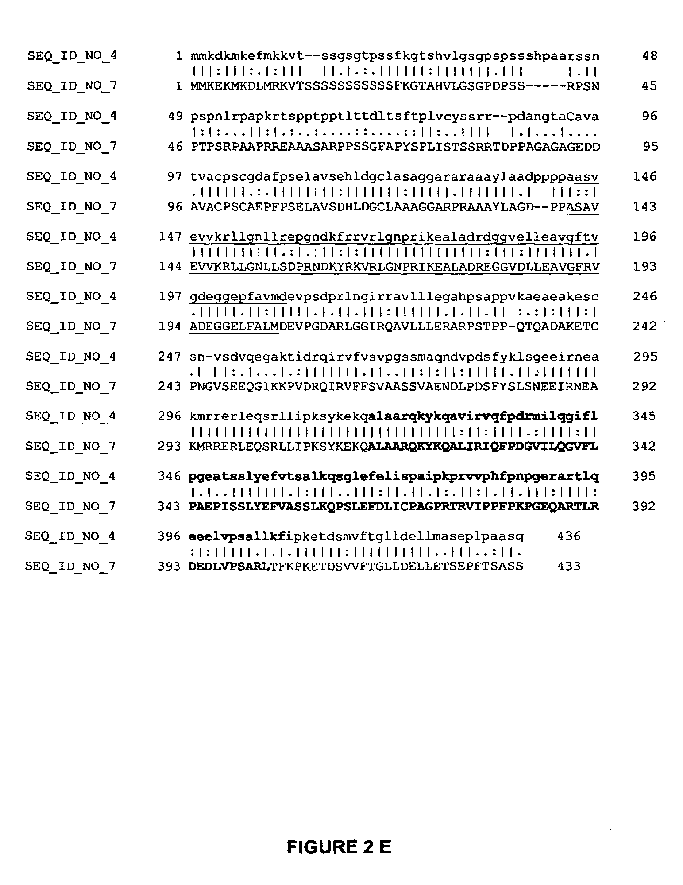

The GRUBX proteins of *Nicotiana tabacum* were analysed with the SMART tool and were used to screen the Pfam (Version 11.0, November 2003; Bateman et al. (2002) Nucl. Acids Res. 30, 276-280) and InterPro database (Release 7.0, 22 July 2003; Mulder et al. (2003) Nucl. Acids. Res. 31, 315-318). GRUBX proteins comprise an UBX domain (PF00789, SM00166, IPR001012) and a PUG domain (SM00580, IPR006567). The UBX domain, as defined in InterPro, is found in ubiquitin-regulatory proteins, which are members of the ubiquitination pathway, as well as a number of other proteins including FAF-1 (FAS-associated factor 1), the human Rep-8 reproduction protein and several hypothetical proteins from yeast. In *Arabidopsis,* there are approximately twenty proteins predicted to comprise this domain. The PUG domain is found in protein kinases, N-glycanases and other nuclear proteins in eukaryotes and is postulated to be involved in protein-protein interactions (for a review see Suzuki & Lennarz (2003) Biochem Biophys Res Commun. 302,1-5 and Biochem Biophys Res Commun. 303, 732) and in RNA binding (Doerks et al., 2002). PUG domains are often found together with UBA or UBX domains in *Arabidopsis* proteins (Doerks et al, 2002). A consensus sequence for the UBX and PUG domains, as defined in the SMART database (Software Version 4.0, sequence database update of 15 September 2003) is given in Figure 2a; Figure 2b shows the UBX and PUG domains of respectively SEQ ID NO 2 and SPTrEMBL Q9ZU93; Figure 2c shows a BLAST alignment of these 2 proteins; and Figures 2d and 2e display an alignment between SEQ ID NO 2 and SEQ ID NO 4, and SEQ ID NO 4 and SEQ ID NO 7, respectively. The PUG and UBX domains are indicated.

Optionally, a zinc finger domain may be present in the GRUBX protein. Zinc finger domains, as defined in InterPro, are nucleic acid-binding protein structures that were first identified in the *Xenopus laevis* transcription factor TFIIIA. These domains have since been found in numerous nucleic acid-binding proteins. A zinc finger domain is composed of 25 to 30 aminoacid residues including 2 conserved Cys and 2 conserved His residues in a C-2-C-12-H-3-H type motif. The 12 residues separating the second Cys and the first His are mainly polar and basic, indicating that this region is involved in nucleic acid binding. The zinc finger motif is an unusually small, self-folding domain in which Zn is a crucial component of its tertiary structure. All Zinc finger domains bind an atom of Zn in a tetrahedral array resulting in the formation of a finger-like projection which may interact with nucleotides in the major groove of the nucleic acid. The Zn binds to the conserved Cys and His residues. Fingers have been found to bind to about 5 base pairs of nucleic acid-containing short runs of guanine residues, and have the ability to bind to both RNA and DNA. The zinc finger may thus represent the original nucleic acid binding protein. It has also been suggested that a Zn-centred domain could be used in a protein interaction, for example in protein kinase C. Many classes of zinc fingers are characterized according to the number and positions of the histidine and cysteine residues involved in the spatial positioning of the zinc atom. In the first class to be characterized, called C2H2 (IPR007087), the first pair of zinc coordinating residues consists of cysteines, while the second pair are histidines. Another Zinc finger domain (IPR006642) may be of the type found in the *Saccharomyces cerevisiae* protein Rad18. Here too, the zinc finger domain is a putative nucleic acid binding sequence. The optional Zinc finger domain in the GRUBX protein as defined herein is however not restricted to the C2H2 or Rad18 type, but can be any type of Zinc finger domain.

The term *GRUBX* nucleic acid/gene, as defined herein, refers to any nucleic acid encoding a GRUBX protein as defined above, or the complement thereof. The nucleic acid may be derived (either directly or indirectly (if subsequently modified)) from any natural or artificial source provided that the nucleic acid, when expressed in a plant, leads to expression of an isolated *GRUBX* nucleic acid/gene. The nucleic acid may be isolated from a microbial source, such as bacteria, yeast or fungi, or from a plant, algal or animal (including human) source. Preferably the nucleic acid is derived from a eukaryotic organism. Preferably the *GRUBX* nucleic acid is of plant origin, further preferably of monocotyledonous or dicotyledonous plant origin, more preferably the *GRUBX* nucleic acid encodes a GRUBX protein from the family of Solanaceae, furthermore preferably the *GRUBX* nucleic acid is a nucleic acid sequence from *Nicotiana tabacum,* most preferably the *GRUBX* nucleic acid is a nucleic acid sequence as represented by SEQ ID NO: 1, and also encompasses nucleic acids encoding an amino acid sequence represented by SEQ ID NO: 2. Alternatively, the nucleic acid encoding a GRUBX protein may be derived from the family of the Poaceae, preferably from *Oryza sativa.* This nucleic acid may be substantially modified from its native form in composition and/or genomic environment through deliberate human manipulation. The nucleic acid sequence is preferably a homologous nucleic acid sequence, i.e. a structurally and/or functionally related nucleic acid sequence, preferably obtained from a plant, whether from the same plant species or different.

The sequence represented by SEQ ID NO: 6 was hitherto unknown.

The sequence represented by SEQ ID NO: 4 was assembled from 4 EST sequences (CA154270, CA144028, BQ535511 & CA184742) and was hitherto unknown. The methods according to the present invention may also be practised by introducing into a plant at least a part of a (natural or artificial) chromosome (such as a Bacterial Artificial Chromosome (BAC)), which chromosome contains at least a gene/nucleic acid sequence encoding a GRUBX protein as defined above (such as SEQ ID NO: 1 or SEQ ID NO 3), preferably together with one or more related gene family members and/or nucleic acid sequence(s) encoding regulatory proteins for GRUBX expression and/or activity. Therefore, according to a further aspect of the present invention, there is provided a method for improving seed yield of plants by introducing into a plant at least a part of a chromosome comprising at least a gene/nucleic acid encoding a GRUBX protein.

According to another aspect of the present invention, advantage may be taken of the nucleic acid encoding a GRUBX protein in breeding programmes. The nucleic acid sequence may be on a chromosome, or a part thereof, comprising at least the nucleic acid sequence encoding the GRUBX protein and preferably also one or more related family members. In an example of such a breeding programme, a DNA marker is identified which may be genetically linked to a gene capable of modulating expression of a nucleic acid encoding a GRUBX protein in a plant, which gene may be a gene encoding the GRUBX protein itself or any other gene which may directly or indirectly influence expression of the gene encoding a GRUBX protein and/or activity of the GRUBX protein itself. This DNA marker may then be used in breeding programs to select plants having improved seed yield.

The present invention therefore extends to the use of a nucleic acid sequence encoding a GRUBX protein in breeding programs.

According to the present invention, enhanced or increased expression of a nucleic acid is envisaged. Methods for obtaining enhanced or increased expression of genes or gene products are well documented in the art and include, for example, overexpression driven by a (strong) promoter, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a *GRUBX* nucleic acid as defined above. For example, endogenous promoters may be altered *in vivo* by mutation, deletion, and/or substitution (see Kmiec, U.S. Pat. No. 5,565,350; Zarling et al., PCT/US93/03868), or isolated promoters may be introduced into a plant cell in the proper orientation and distance from a gene of the present invention so as to control the expression of the gene. Preferably, the nucleic acids useful in the present invention are overexpressed in a plant or plant cell. The term overexpression as used herein means any form of expression that is additional to the original wild-type expression level. Preferably the nucleic acid to be introduced into the plant and/or the nucleic acid that is to be overexpressed in the plants is in a sense direction with respect to the promoter to which it is operably linked. Preferably, the nucleic acid to be overexpressed encodes a GRUBX protein, further preferably the nucleic acid sequence encoding the GRUBX protein is isolated from a dicotyledonous plant, preferably of the family Solanaceae, further preferably wherein the sequence is isolated from *Nicotiana tabacum,* most preferably the nucleic acid sequence is as represented by SEQ ID NO: 1, or encodes an amino acid sequence as represented by SEQ ID NO: 2. Alternatively, the nucleic acid sequence encoding the GRUBX protein is as represented by MIPS No. At2g01650, SEQ ID NO: 3 or 6, or encodes an amino acid sequence as represented by Q9ZU93, SEQ ID NO: 4 or 7. It should be noted that the applicability of the invention does not rest upon the use of the nucleic acid represented by SEQ ID NO: 1, nor upon the nucleic acid sequence encoding the amino acid sequence of SEQ ID NO: 2, but that other nucleic acid sequences may be used in the methods of the present invention. In particular, the nucleic acids useful in the methods of the present invention encode proteins having at least 80% sequence identity to any one of SEQ ID NO: 2, 4, 7, said polypeptide having ubiquitin-regulatory action within the ubiquitination pathway , and optionally also a Zinc finger domain.

Plants are transformed with a vector comprising the sequence of interest (i.e., the nucleic acid sequence capable of modulating expression of nucleic acid encoding a GRUBX protein), which sequence is operably linked to one or more control sequences (at least a promoter). The terms "regulatory element", "control sequence" and "promoter" are all used herein interchangeably and are to be taken in a broad context to refer to regulatory nucleic acid sequences capable of effecting expression of the sequences to which they are ligated. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative which confers, activates or enhances expression of a nucleic acid molecule in a cell, tissue or organ. The term "operably linked" as used herein refers to a functional linkage between the promoter sequence and the gene of interest, such that the promoter sequence is able to initiate transcription of the gene of interest. Advantageously, any type of promoter may be used to drive expression of the nucleic acid sequence depending on the desired outcome. Suitable promoters include promoters that are active in monocotyledonous plants such as rice or maize.

The nucleic acid sequence encoding a GRUBX protein is operably linked to a seed-preferred promoter. The term "seed-preferred" as defined herein refers to a promoter that is expressed predominantly in seed tissue, but not necessarily exclusively in this tissue. The term "seed-preferred" encompasses all promoters that are active in seeds. Seed tissue encompasses any part of the seed including the endosperm, aleurone or embryo. Preferably, the seed-preferred promoter is a prolamin promoter, or a promoter of similar strength and/or a promoter with a similar expression pattern. Most preferably, the prolamin promoter is as represented by nucleotides 1-654 in the expression cassette of SEQ ID NO: 5. Promoter strength and/or expression pattern can be analysed for example by coupling the promoter to a reporter gene and assay the expression of the reporter gene in various tissues of the plant. One suitable reporter gene well known to a person skilled in the art is bacterial *beta-glucuronidase.* Examples of other seed-preferred promoters are presented in Table 1, and these promoters are useful for the methods of the present invention.

**TABLE 1: Examples of seed-preferred promoters for use in the performance of the present invention:**

| GENE SOURCE | EXPRESSION PATTERN | REFERENCE |
|---|---|---|
| seed-specific genes | seed | Simon, et al., Plant Mol. Biol. 5: 191, 1985; Scofield, et al., J. Biol. Chem. 262: 12202, 1987.; Baszczynski, et al., Plant Mol. Biol. 14: 633, 1990. |
| Brazil Nut albumin | seed | Pearson, et al., Plant Mol. Biol. 18: 235-245, 1992. |
| legumin | seed | Ellis, et al., Plant Mol. Biol. 10: 203-214, 1988. |
| glutelin (rice) | seed | Takaiwa, et al., Mol. Gen. Genet. 208: 15-22, 1986; Takaiwa, et al., FEBS Letts. 221: 43-47, 1987. |
| zein | seed | Matzke et al Plant Mol Biol, 14(3):323-32 1990 |
| napA | seed | Stalberg, et al, Planta 199: 515-519, 1996. |
| wheat LMW and HMW glutenin-1 | endosperm | Mol Gen Genet 216:81-90, 1989; NAR 17:461-2, 1989 |
| wheat SPA | seed | Albani et al, Plant Cell, 9: 171-184, 1997 |
| wheat α,β,γ-gliadins | endosperm | EMBO J. 3:1409-15, 1984 |
| barley *Itr1* promoter | endosperm | |
| barley B1, C, D, hordein | endosperm | Theor Appl Gen 98:1253-62, 1999; Plant J 4:343-55, 1993; Mol Gen Genet 250:750-60, 1996 |
| barley DOF | endosperm | Mena et al, The Plant Journal, 116(1): 53-62, 1998 |
| *blz2* | endosperm | EP99106056.7 |
| synthetic promoter | endosperm | Vicente-Carbajosa et al., Plant J. 13: 629-640, 1998. |
| rice prolamin NRP33 | endosperm | Wu et al, Plant Cell Physiology 39(8) 885-889, 1998 |
| rice α-globulin Glb-1 | endosperm | Wu et al, Plant Cell Physiology 39(8) 885-889, 1998 |
| rice OSH1 | embryo | Sato *et al,* Proc. Natl. Acad. Sci. USA, 93: 8117-8122, 1996 |
| rice α-globulin REB/OHP-1 | endosperm | Nakase et al. Plant Mol. Biol. 33: 513-522, 1997 |
| rice ADP-glucose PP | endosperm | Trans Res 6:157-68, 1997 |
| maize ESR gene family | endosperm | Plant J 12:235-46, 1997 |
| sorgum γ-kafirin | endosperm | PMB 32:1029-35, 1996 |
| KNOX | embryo | Postma-Haarsma et al, Plant Mol. Biol. 39:257-71, 1999 |
| rice oleosin | embryo and aleuron | Wu et at, J. Biochem., 123:386, 1998 |
| sunflower oleosin | seed (embryo and dry seed) | Cummins, *et al.,* Plant Mol. Biol. 19: 873-876, 1992 |
| PRO0117, putative rice 40S ribosomal protein | weak in endosperm | WO2004/070039 |
| PRO0135, rice alpha-globulin | strong in endosperm | |
| PRO013 rice alanine aminotransferase | weak in endosperm | |
| PRO0147 trypsin inhibitor ITR1 (barley) | weak in endosperm | |
| PRO0151, rice WSI18 | embryo + stress | WO2004/0700 |
| PRO017 rice RAB21 | embryo + stress | WO2004/070039 |
| PRO0218, rice oleosin 18kd | aleurone + embryo | |

An intron sequence may also be added to the 5' untranslated region or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg, Mol. Cell Biol. 8, 4395-4405 (1988); Callis et al., Genes Dev. 1, 1183-1200 (1987)). Such intron enhancement of gene expression is typically greatest when placed near the 5' end of the transcription unit. Use of the maize introns Adh1-S intron 1, 2, and 6, the Bronze-1 intron are known in the art. See generally, The Maize Handbook, Chapter 116, Freeling and Walbot, Eds., Springer, N.Y. (1994).

The GRUBX protein itself and/or the *GRUBX* nucleic acid itself may be introduced directly into a plant cell or into the plant itself (including introduction into a tissue, organ or any other part of the plant). According to a preferred feature of the present invention, the nucleic acid is preferably introduced into a plant by transformation. The nucleic acid is preferably as represented by SEQ ID NO: 1, or is a nucleic acid encoding an amino acid sequence represented by SEQ ID NO: 2. Alternatively, the nucleic acid sequence is as represented by any of MIPS No. At2g01650, SEQ ID NO: 3, SEQ ID NO 6. The amino acid sequence may alternatively be a sequence as represented by any of SPTrEMBL Q9ZU93, GenBank Acc. Nr. AAR01744, SEQ ID NO: 4, SEQ ID NO 7.

The term "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated therefrom. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (for example, apical meristem, axillary buds, and root meristems), and induced meristem tissue (for example, cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell can then be used to regenerate a transformed plant in a manner known to persons skilled in the art.

Transformation of a plant species is now a fairly routine technique. Advantageously, any of several transformation methods may be used to introduce the gene of interest into a suitable ancestor cell. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., 1882, Nature 296, 72-74; Negrutiu I. et al., June 1987, Plant Mol. Biol. 8, 363-373); electroporation of protoplasts (Shillito R.D. et al., 1985 Bio/Technol 3, 1099-1102); microinjection into plant material (Crossway A. et al., 1986, Mol. Gen Genet 202, 179-185); DNA or RNA-coated particle bombardment (Klein T.M. et al., 1987, Nature 327, 70) infection with (non-integrative) viruses and the like. Transgenic rice plants overexpressing a *GRUBX* gene are preferably produced via Agrobacterium-mediated transformation using any of the well known methods for rice transformation, such as described in any of the following: published European patent application EP 1198985 A1, Aldemita and Hodges (Planta, 199, 612-617, 1996); Chan et al. (Plant Mol. Biol. 22 (3) 491-506, 1993), Hiei et al. (Plant J. 6 (2) 271-282, 1994). In the case of corn transformation, the preferred method is as described in either Ishida et al. (Nat. Biotechnol. 1996 Jun; 14(6): 745-50) or Frame et al. (Plant Physiol. 2002 May; 129(1): 13-22).

Generally after transformation, plant cells or cell groupings are selected for the presence of one or more markers which are encoded by plant-expressible genes co-transferred with the gene of interest, following which the transformed material is regenerated into a whole plant.

Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed to give homozygous second-generation (or T2) transformants, and the T2 plants further propagated through classical breeding techniques.

The generated transformed organisms may take a variety of forms. For example, they may be chimeras of transformed cells and non-transformed cells; clonal transformants (for example, all cells transformed to contain the expression cassette); grafts of transformed and untransformed tissues (for example, in plants, a transformed rootstock grafted to an untransformed scion). The present invention clearly extends to any plant cell or plant produced by any of the methods described herein, and to all plant parts, propagules and progeny thereof. The term "plant" as used herein encompasses whole plants, ancestors and progeny of the plants, plant parts, plant cells, tissues and organs. The term "plant" also therefore encompasses suspension cultures, embryos, meristematic regions, callus tissue, leaves, flowers, fruits, seeds, roots (including rhizomes and tubers), shoots, bulbs, stems, gametophytes, sporophytes, pollen, and microspores. Plants that are particularly useful in the methods of the invention include algae, ferns, and all plants which belong to the superfamily Viridiplantae, in particular monocotyledonous and dicotyledonous plants, including fodder or forage legumes, ornamental plants, food crops, trees, or shrubs selected from the list comprising *Abelmoschus* spp., *Acer* spp., *Actinidia* spp., *Agropyron* spp., *Allium* spp., *Amaranthus* spp., *Ananas comosus, Annona* spp., *Apium graveolens, Arabidopsis thaliana, Arachis* spp, *Artocarpus* spp., *Asparagus officinalis, Avena sativa, Averrhoa carambola, Benincasa hispida, Bertholletia excelsea, Beta vulgaris, Brassica* spp., *Cadaba farinosa, Camellia sinensis, Canna indica, Capsicum* spp., *Carica papaya, Carissa macrocarpa, Carthamus tinctorius, Carya* spp., *Castanea* spp., *Cichorium endivia, Cinnamomum spp., Citrullus lanatus, Citrus* spp., *Cocos* spp., *Coffea* spp., *Cola* spp., *Colocasia esculenta, Corylus* spp., *Crataegus* spp., *Cucumis* spp., *Cucurbita* spp., *Cynara* spp., *Daucus carota, Desmodium* spp., *Dimocarpus longan, Dioscorea* spp., *Diospyros* spp., *Echinochloa* spp., *Eleusine coracana, Eriobotrya japonica, Eugenia uniflora, Fagopyrum* spp., *Fagus* spp., *Ficus carica, Fortunella spp., Fragaria* spp., *Ginkgo biloba, Glycine* spp., *Gossypium hirsutum, Helianthus* spp., *Hibiscus* spp., *Hordeum* spp., *Ipomoea batatas, Juglans* spp., *Lactuca sativa, Lathyrus* spp., *Lemna spp., Lens culinaris, Linum usitatissimum, Litchi chinensis, Lotus* spp., *Luffa acutangula, Lupinus* spp., *Macrotyloma* spp., *Malpighia emarginata, Malus* spp., *Mammea americana, Mangifera indica, Manihot* spp., *Manilkara zapota, Medicago sativa, Melilotus* spp., *Mentha* spp., *Momordica* spp., *Morus nigra, Musa* spp., *Nicotiana* spp., Olea spp., *Opuntia* spp., *Ornithopus* spp., *Oryza* spp., *Panicum miliaceum, Passiflora edulis, Pastinaca sativa, Persea* spp., *Petroselinum crispum, Phaseolus* spp., *Phoenix* spp., *Physalis* spp., *Pinus* spp., *Pistacia vera, Pisum* spp., Poa spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Rubus* spp., *Saccharum* spp., *Sambucus* spp., *Secale cereale, Sesamum* spp., *Solanum* spp., *Sorghum bicolor, Spinacia* spp., *Syzygium* spp., *Tamarindus indica, Theobroma cacao, Trifolium* spp., *Triticosecale rimpaui, Triticum* spp., *Vaccinium* spp., *Vicia* spp., *Vigna spp., Vitis* spp., *Zea mays, Zizania palustris, Ziziphus* spp., amongst others.

According to a preferred feature of the present invention, the plant is a crop plant comprising soybean, sunflower, canola, alfalfa, rapeseed or cotton. Further preferably, the plant according to the present invention is a monocotyledonous plant such as sugarcane, most preferably a cereal, such as rice, maize, wheat, millet, barley, rye, sorghum or oats. However, it is envisaged that the methods of the present invention can be applied to a wide variety of plants, since the domain conservation among the known eukaryotic GRUBX homologues suggests an equally conserved function in cellular metabolism.

Advantageously, performance of the methods according to the present invention results in plants having improved seed yield relative to corresponding wild type plants.

The present invention relates to methods to improve harvest index.

The improved harvest index is expressed as a ratio of the total biomass over the yield of harvestable parts, such as seeds; or Thousand Kernel Weight. According to the present invention, performance of the methods according to the present invention result in plants having an increase in seed yield, relative to control plants. Therefore, according to the present invention, there is provided a method for increasing seed yield, which method comprises introducing and overexpressing a nucleic acid sequence encoding a GRUBX protein as defined above, preferably wherein the GRUBX protein is encoded by a nucleic acid sequence represented by SEQ ID NO: 1 or wherein the GRUBX protein is represented by SEQ ID NO: 2. Alternatively, the GRUBX may be encoded by a nucleic acid sequence represented by any of MIPS No. At2g01650, SEQ ID NO: 3, or wherein the GRUBX is represented by any of SPTrEMBL Q9ZU93, SEQ ID NO: 4.

The methods of the present invention are favourable to apply to crop plants because the methods of the present invention are used to increase the seed yield of a plant. Therefore, the methods of the present invention are particularly useful for crop plants cultivated for their seeds, such as cereals. Accordingly, a particular embodiment of the present invention relates to a method to increase the seed yield of a cereal.

An increase in yield and/or growth occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Due to advances in agricultural practices (irrigation, fertilisation, pesticide treatments) severe stresses are not often encountered in cultivated crop plants. As a consequence, the compromised growth induced by mild stress is often an undesirable feature for agriculture. Mild stresses are the typical stresses to which a plant may be exposed. These stresses may be the everyday biotic and/or abiotic (environmental) stresses to which a plant is exposed. Typical abiotic or environmental stresses include temperature stresses caused by atypical hot or cold/freezing temperatures, salt stress, water stress (drought or excess water). Abiotic stresses may also be caused by chemicals. Biotic stresses are typically those stresses caused by pathogens, such as bacteria, viruses, fungi or insects.

The present invention also relates to the use of a nucleic acid encoding a GRUBX protein in improving seed yield of plants. The present invention also relates to the use of a GRUBX protein in improving seed yield of plants. The nucleic acid sequence is preferably as represented by SEQ ID NO: 1, 3, or 6, or encodes an amino acid sequence represented by SEQ ID NO: 2, 4, 7.

The present invention will now be described with reference to the following figures in which:
**Figure 1a****.** Phylogenetic tree representing *Arabidopsis thaliana* proteins and animal reference proteins comprising an UBX domain, as recognised by the SMART tool. The human proteins are represented by their GenBank Accession numbers NP_079517 *(Homo sapiens* UBX domain containing 1 (UBXD1)), AAP97263 *(Homo sapiens* Fas-associated protein factor FAF1 mRNA), NP_005662 *(Homo sapiens* reproduction 8 (D8S2298E), REP8) and a rat protein by NP_114187 *(Rattus norvegicus* p47 protein). The other identifiers (except for SEQ ID NO 2, SEQ ID NO 4 and SEQ ID NO 7) are GenBank or SPTrEMBL accession numbers for *Arabidopsis thaliana* proteins.
**Figure 1b****.** Phylogenetic tree representing plant proteins comprising a PUG domain, as recognised by the SMART tool. SEQ ID NO 2 and SEQ ID NO 4 are compared with *Arabidopsis thaliana* proteins (SPTrEMBL accessions Q9ZU93 (Expressed protein), Q9FKI1 (Similarity to zinc metalloproteinase), Q9MAT3 (F13M7.16 protein), Q9FKC7 (Genomic DNA, chromosome 5, TAC clone:K24G6), Q9SF12 (Hypothetical protein), Q9C5S2 (Endoribonuclease/protein kinase IRE1), Q8RX75 (AT5g24360/K16H17_7), Q94lG5 (Ire1 homolog-1)), and with the rice protein SPTrEMBL Q7XIT1 (Oslre1p).
**Figure 2a****.** Definition of UBX1 and PUG domains by their consensus sequences (SMART database). CONSENSUS/50%, respectively /65% and /80% are the consensus sequences for the top 50, 65 and 80% of the reference sequences comprising the UBX1 or PUG domain. The capital letters are the standard single letter IUPAC codes for the various amino acids, the other letters symbolise the nature of the amino acids as outlined below:

| Class | Key | Residues |
|---|---|---|
| Alcohol | o | S,T |
| Aliphatic | I | I,L,V |
| Any | . | A,C,D,E,F,G,H,I,K,L,M,N,P,Q,R,S,T,V,W,Y |
| Aromatic | a | F,H,W,Y |
| Charged | c | D,E,H,K,R |
| Hydrophobic | h | A,C,F,G,H,I,K,L,M,R,T,V,W,Y |
| Negative | - | D,E |
| Polar | p | C,D,E,H,K,N,Q,R,S,T |
| Positive | + | H,K,R |
| Small | s | A,C,D,G,N,P,S,T,V |
| Tiny | u | A,G,S |
| Turnlike | t | A,C,D,E,G,H,K,N,Q,R,S,T |

**Figure 2b****.** UBX and PUG domain sequences present in SEQ ID NO 2 and in Q9ZU93.
**Figure 2c****.** Alignment of Q9ZU93 and SEQ ID NO 2, PUG domains underlined, UBX domains in bold.
**Figure 2d****.** Alignment of SEQ ID NO 2 and SEQ ID NO 4, PUG domains underlined, UBX domains in bold.
**Figure 2e****.** Alignment of SEQ ID NO 4 and SEQ ID NO 7, PUG domains underlined, UBX domains in bold.
**Figure 3****.** Schematic presentation of the entry clone p77, containing CDS0669 within the AttL1 and AttL2 sites for Gateway® cloning in the pDONR201 backbone. CDS0669 is the internal code for the tobacco *GRUBX* coding sequence. This vector contains also a bacterial kanamycin-resistance cassette and a bacterial origin of replication.

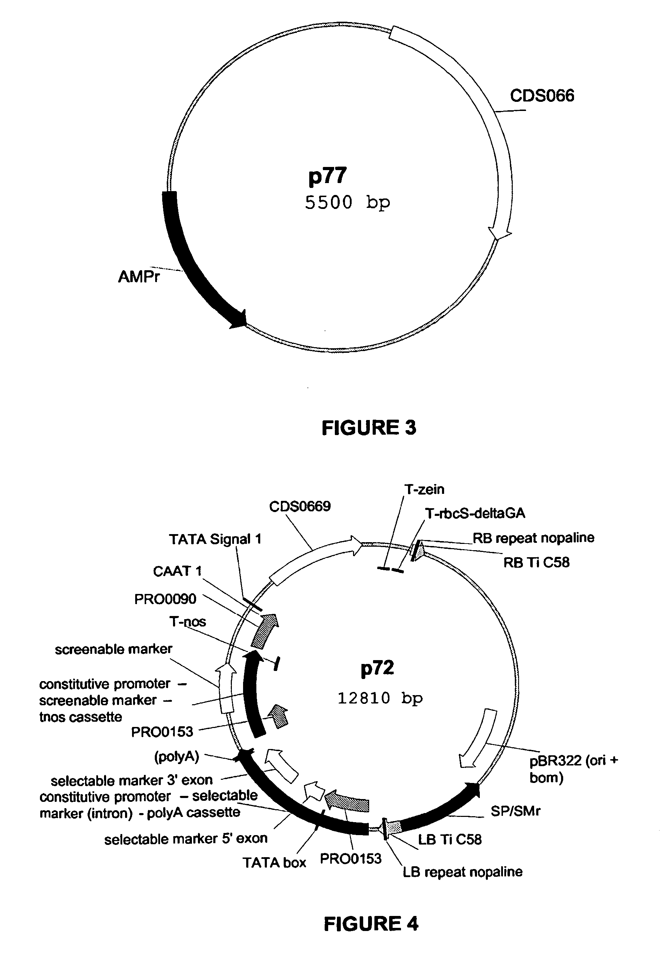

**Figure 4****.** Binary vector for the expression in *Oryza sativa* of the tobacco GRUBX gene (CDS0669) under the control of the prolamin promoter (PRO0090) This vector contains a T-DNA derived from the Ti Plasmid, limited by a left border (LB repeat, LB Ti C58) and a right border (RB repeat, RB Ti C58)). From the left border to the right border, this T-DNA contains: a cassette for antibiotic selection of transformed plants; a cassette for visual screening of transformed plants; the PRO0090 CDS0669 -zein and rbcS-deltaGA double terminator cassette for expression of the tobacco *GRUBX* gene. This vector also contains an origin of replication from pBR322 for bacterial replication and a selectable marker (Spe/SmeR) for bacterial selection with spectinomycin and streptomycin.

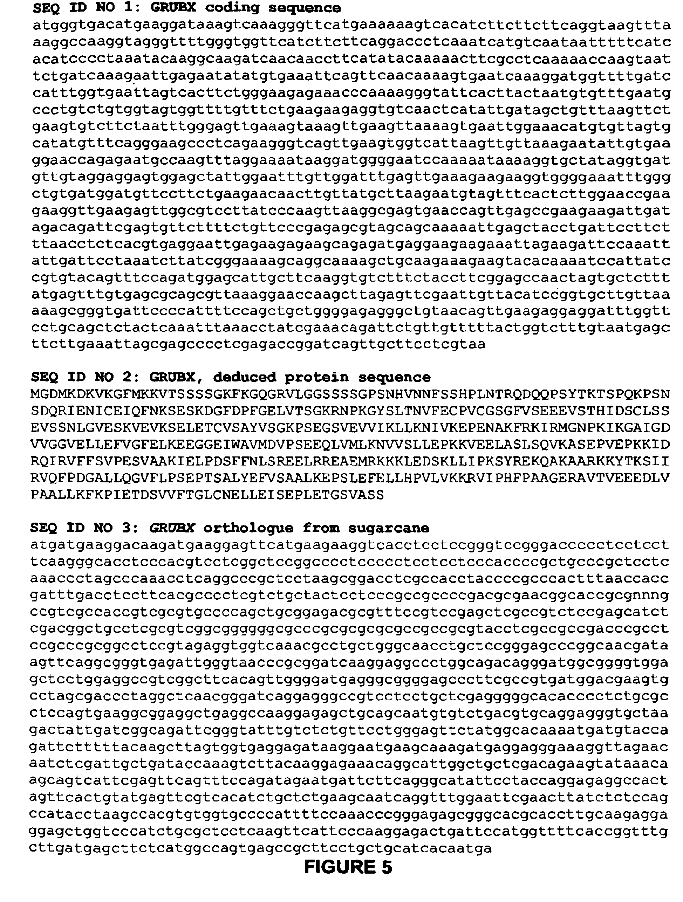

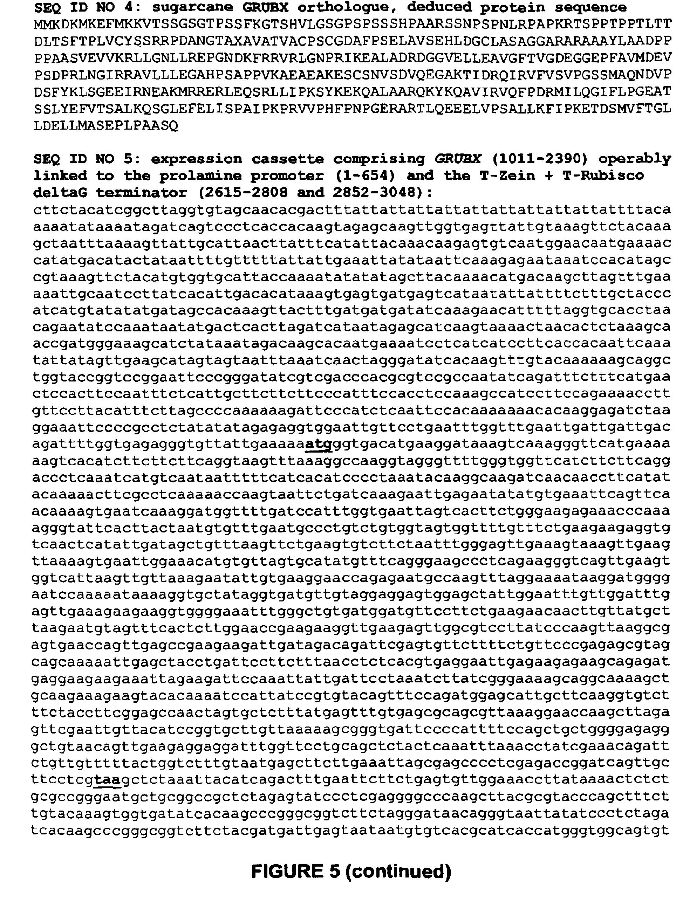

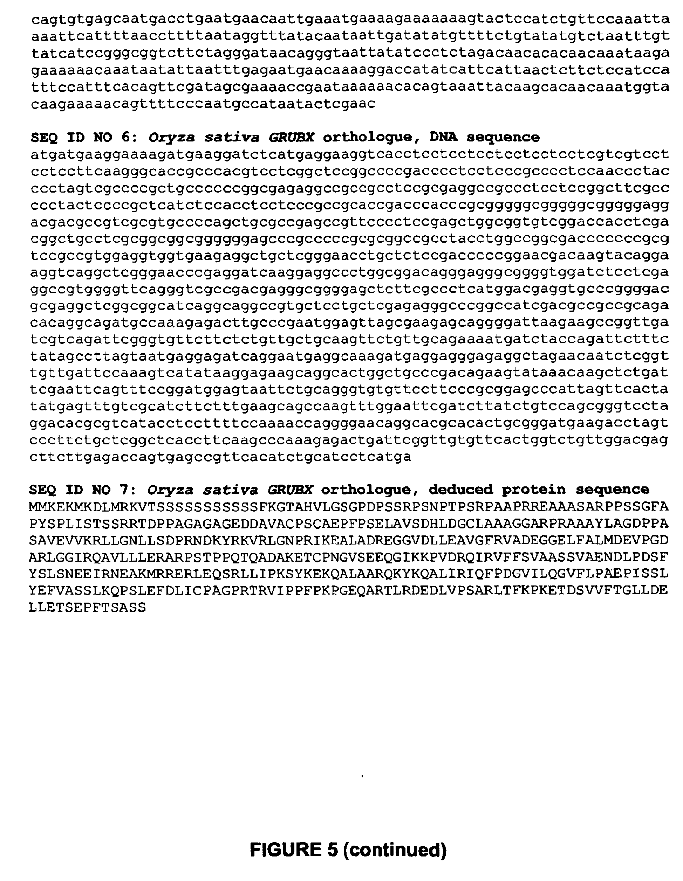

**Figure 5****.** Examples of sequences useful in the present invention. SEQ ID NO: 1 and SEQ ID NO: 2 are the sequences of the *GRUBX* nucleic acid and GRUBX protein respectively that were used in the examples. SEQ ID NO: 3 and SEQ ID NO: 4 represent the coding sequence and the protein sequence of the sugarcane GRUBX orthologue, SEQ ID NO: 5 is the sequence of the expression cassette that was used in the transformed rice plants, SEQ ID NO: 6 and SEQ ID NO: 7 represent the encoding sequence respectively protein sequence of the rice GRUBX orthologue.

### Examples

The present invention will now be described with reference to the following examples, which are by way of illustration alone.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (Current Protocols in Molecular Biology. New York: John Wiley and Sons, 1998). Standard materials and methods for plant molecular work are described in Plant Molecular Biology Labfax (1993) by R.D.D. Croy, published by BIOS Scientific Publications Ltd (UK) and Blackwell Scientific Publications (UK).

### Example 1: Cloning of the CDS0669 sequence

### Cloning of the GRUBX gene fragment from tobacco

A cDNA-AFLP experiment was performed on a synchronized tobacco BY2 cell culture *(Nicotiana tabacum* L. cv. Bright Yellow-2), and BY2 expressed sequence tags that were cell cycle modulated were elected for further cloning. The expressed sequence tags were used to screen a tobacco cDNA library and to isolate the full-length cDNA of interest, namely one coding for *GRUBX gene* (CDS0669).

### Synchronization of BY2 cells.

A tobacco BY2 *(Nicotiana tabacum* L. cv. Bright Yellow-2) cultured cell suspension was synchronized by blocking cells in early S-phase with aphidicolin as follows. The cell suspension of *Nicotiana tabacum* L. cv. Bright Yellow 2 was maintained as described (Nagata et al. Int. Rev. Cytol. 132, 1-30, 1992). For synchronization, a 7-day-old stationary culture was diluted 10-fold in fresh medium supplemented with aphidicolin (Sigma-Aldrich, St. Louis, MO; 5 mg/l), a DNA-polymerase α inhibiting drug. After 24 h, cells were released from the block by several washings with fresh medium after which their cell cycle progression resumed.

### RNA extraction and cDNA synthesis.

Total RNA was prepared using LiCl precipitation (Sambrook et al, 2001) and poly(A⁺) RNA was extracted from 500 µg of total RNA using Oligotex columns (Qiagen, Hilden, Germany) according to the manufacturer's instructions. Starting from 1 µg of poly(A⁺) RNA, first-strand cDNA was synthesized by reverse transcription with a biotinylated oligo-dT₂₅ primer (Genset, Paris, France) and Superscript II (Life Technologies, Gaithersburg, MD). Second-strand synthesis was done by strand displacement with *Escherichia coli* ligase (Life Technologies), DNA polymerase I*(USB, Cleveland, OH) and RNAse-H (USB).

### cDNA-AFLP analysis.

Five hundred ng of double-stranded cDNA was used for AFLP analysis as described (Vos et al., Nucleic Acids Res. 23 (21) 4407-4414, 1995; Bachem et al., Plant J. 9 (5) 745-53, 1996) with modifications. The restriction enzymes used were *Bst*YI and *Mse*I (Biolabs) and the digestion was done in two separate steps. After the first restriction digest with one of the enzymes, the 3' end fragments were trapped on Dyna beads (Dynal, Oslo, Norway) by means of their biotinylated tail, while the other fragments were washed away. After digestion with the second enzyme, the released restriction fragments were collected and used as templates in the subsequent AFLP steps. For pre-amplifications, a *Msi*I primer without selective nucleotides was combined with a *Bst*YI primer containing either a T or a C as 3' most nucleotide. PCR conditions were as described (Vos et al., 1995). The obtained amplification mixtures were diluted 600-fold and 5 µl was used for selective amplifications using a P³³-labeled *Bst*YI primer and the Amplitaq-Gold polymerase (Roche Diagnostics, Brussels, Belgium). Amplification products were separated on 5% polyacrylamide gels using the Sequigel system (Biorad). Dried gels were exposed to Kodak Biomax films as well as scanned in a Phosphorlmager (Amersham Pharmacia Biotech, Little Chalfont, UK).

### Characterization of AFLP fragments.

Bands corresponding to differentially expressed transcripts, among which the (partial) transcript corresponding to SEQ ID NO 1 (or CDS0669), were isolated from the gel and eluted DNA was re-amplified under the same conditions as for selective amplification. Sequence information was obtained either by direct sequencing of the re-amplified polymerase chain reaction product with the selective *Bst*YI primer or after cloning the fragments in pGEM-T easy (Promega, Madison, WI) and sequencing of individual clones. The obtained sequences were compared against nucleotide and protein sequences present in the publicly available databases by BLAST sequence alignments (Altschul et al., Nucleic Acids Res. 25 (17) 3389-3402 1997). When available, tag sequences were replaced with longer EST or isolated cDNA sequences to increase the chance of finding significant homology. The physical cDNA clone corresponding to SEQ ID NO 1 (CDS0669) was subsequently amplified from a commercial tobacco cDNA library as follows:

### Cloning of the GRUBX gene (CDS0669)

A c-DNA library with an average size of inserts of 1,400 bp was prepared from poly(A⁺) RNA isolated from actively dividing, non-synchronized BY2 tobacco cells. These library-inserts were cloned in the vector pCMVSPORT6.0, comprising an attB Gateway cassette (Life Technologies). From this library, 46,000 clones were selected, arrayed in 384-well microtiter plates, and subsequently spotted in duplicate on nylon filters. The arrayed clones were screened using pools of several hundreds of radioactively labelled tags as probes (including the BY2-tag corresponding to the sequence CDS0669, SEQ ID NO 1). Positive clones were isolated (among which the clone corresponding to CDS0669, SEQ I NO 1), sequenced, and aligned with the tag sequence. Where the hybridisation with the tag failed, the full-length cDNA corresponding to the tag was selected by PCR amplification: tag-specific primers were designed using primer3 program (http://www-genome.wi.mit.edu/genome software/other/primer3.html) and used in combination with a common vector primer to amplify partial cDNA inserts. Pools of DNA from 50,000, 100,000, 150,000, and 300,000 cDNA clones were used as templates in the PCR amplifications. Amplification products were then isolated from agarose gels, cloned, sequenced and their sequence aligned with those of the tags. Next, the full-length cDNA corresponding to the nucleotide sequence of SEQ ID NO 1 was cloned from the pCMVsport6.0 library vector into pDONR201, a Gateway^{®} donor vector (Invitrogen, Paisley, UK) via a LR reaction, resulting in the entry clone p77 (Figure 3).

### Example 2: Vector construction

The entry clone p77 was subsequently used in an LR reaction with p0830, a destination vector used for *Oryza sativa* transformation. This vector contained as functional elements within the T-DNA borders: a plant selectable marker; a visual marker expression cassette; and a Gateway cassette intended for LR *in vivo* recombination with the sequence of interest already cloned in the entry clone. A prolamin promoter for seed-preferred expression (PRO0090 was upstream of this Gateway cassette. After the LR recombination step, the resulting expression vector p72 (Figure 4) was transformed into *Agrobacterium* strain LBA4404 and subsequently into *Oryza sativa* plants.

### Example 3: Transformation of rice with the PRO0090-CDS0669 construct

Mature dry seeds of *Oryza sativa* japonica cultivar Nipponbare were dehusked. Sterilization was done by incubating the seeds for one minute in 70% ethanol, followed by 30 minutes in 0.2% HgCl₂ and by 6 washes of 15 minutes with sterile distilled water. The sterile seeds were then germinated on a medium containing 2,4-D (callus induction medium). After a 4-week incubation in the dark, embryogenic, scutellum-derived calli were excised and propagated on the same medium. Two weeks later, the calli were multiplied or propagated by subculture on the same medium for another 2 weeks. 3 days before co-cultivation, embryogenic callus pieces were sub-cultured on fresh medium to boost cell division activity. The *Agrobacterium* strain LBA4414 harbouring binary vector p72 was used for co-cultivation. The *Agrobacterium* strain was cultured for 3 days at 28°C on AB medium with the appropriate antibiotics. The bacteria were then collected and suspended in liquid co-cultivation medium at an OD₆₀₀ of about 1. The suspension was transferred to a petri dish and the calli were immersed in the suspension for 15 minutes. Next, the callus tissues were blotted dry on a filter paper, transferred to solidified co-cultivation medium and incubated for 3 days in the dark at 25°C. Thereafter, co-cultivated callus was grown on 2,4-D-containing medium for 4 weeks in the dark at 28°C in the presence of a selective agent at a suitable concentration. During this period, rapidly growing resistant callus islands developed. Upon transfer of this material to a regeneration medium and incubation in the light, the embryogenic potential was released and shoots developed in the next four to five weeks. Shoots were excised from the callus and incubated for 2 to 3 weeks on an auxin-containing medium from which they were transferred to soil. Hardened shoots were grown under high humidity and short days in a greenhouse. Finally seeds were harvested three to five months after transplanting. The method yielded single locus transformants at a rate of over 50 % (Aldemita and Hodges, Planta 199, 612-617, 1996; Chan et al., Plant Mol. Biol. 22(3), 491-506, 1993; Hiei et al., Plant J. 6(2), 271-282, 1994).

### Example 4: Evaluation of transgenic rice transformed with the PRO0090-CDS0669 construct

Approximately 15 to 20 independent T0 rice transformants were generated. The primary transformants were transferred from tissue culture chambers to a greenhouse for growing and harvest of T1 seed. 6 events, of which the T1 progeny segregated 3:1 for presence/absence of the transgene, were retained. For each of these events, approximately 10 T1 seedlings containing the transgene (hetero- and homo-zygotes), and approximately 10 T1 seedlings lacking the transgene (nullizygotes), were selected by monitoring visual marker expression. A number of parameters related to vegetative growth and seed production were evaluated and all data were statistically analysed as outlined below:

### Statistical analysis: t-test and F-test:

A two factor ANOVA (analysis of variants) was used as statistical model for the overall evaluation of plant phenotypic characteristics. An F-test was carried out on all the parameters measured of all the plants of all the events transformed with the gene of the present invention. The F-test is carried out to check for an effect of the gene over all the transformation events and to verify for an overall effect of the gene, also named herein "global gene effect". If the value of the F-test shows that the data are significant, than it is concluded that there is a "gene" effect, meaning that not only presence or the position of the gene is causing the differences in phenotype. The threshold for significance for a true global gene effect is set at 5% probability level for the F-test.

### 4.1 Vegetative growth measurements:

The selected T1 plants (approximately 10 with the transgene and approximately 10 without the transgene) were transferred to a greenhouse. Each plant received a unique barcode label to link unambiguously the phenotyping data to the corresponding plant. The selected T1 plants were grown on soil in 10 cm diameter pots under the following environmental settings: photoperiod= 11.5 h, daylight intensity= 30,000 lux or more, daytime temperature= 28°C or higher, night time temperature= 22°C, relative humidity= 60-70%. Transgenic plants and the corresponding nullizygotes were grown side-by-side at random positions. From the stage of sowing until the stage of maturity each plant was passed several times through a digital imaging cabinet and imaged. At each time point digital images (2048x1536 pixels, 16 million colours) were taken of each plant from at least 6 different angles. Several parameters can be derived in an automated way from all the digital images of all the plants, using image analysis software.

### 4.2 Seed-related parameter measurements:

The mature primary panicles were harvested, bagged, barcode-labelled and then dried for three days in the oven at 37°C. The panicles were then threshed and all the seeds were collected and counted. The filled husks were separated from the empty ones using an airblowing device. The empty husks were discarded and the remaining fraction was counted again. The filled husks were weighed on an analytical balance. This procedure allows to derive a set of seed-related parameters.

### Harvest index of plants

The harvest index in the present invention is defined as the ratio between the total seed yield and the above ground area (mm²), multiplied by a factor 10⁶. The total seed yield per plant was measured by weighing all filled husks harvested from a plant as described above. Plant aboveground area was determined by counting the total number of pixels of the digital images from aboveground plant parts discriminated from the background. This value was averaged for the pictures taken on the same time point from the different angles and was converted to a physical surface value expressed in square mm by calibration. Experiments showed that the aboveground plant area measured this way correlates with the biomass of plant parts above ground.

The data obtained in the first experiment were confirmed in a second experiment with T2 plants. Three lines that had the correct expression pattern were selected for further analysis. Seed batches from the positive plants (both hetero- and homozygotes) in T1, were screened by monitoring marker expression. For each chosen event, the heterozygote seed batches were then retained for T2 evaluation. Within each seed batch an equal number of positive and negative plants were grown in the greenhouse for evaluation.

A total number of 120 *GRUBX* transformed plants were evaluated in the T2 generation, that is 40 plants per event of which 20 positives for the transgene, and 20 negatives.

Because two experiments with overlapping events have been carried out, a combined analysis was performed. This is useful to check consistency of the effects over the two experiments, and if this is the case, to accumulate evidence from both experiments in order to increase confidence in the conclusion. The method used was a mixed-model approach that takes into account the multilevel structure of the data (i.e. experiment - event - segregants). P-values are obtained by comparing likelihood ratio test to chi square distributions.

In a first experiment, six lines in T1 generation were evaluated. There was an average increase of the harvest index and two lines had a significant increase of 50% or more compared to the nullizygote lines (Table 2).

**Table 2: Evaluation of the two best performing T1 events**

| Harvest index : | | | | | |
|---|---|---|---|---|---|
| Line | TR | null | dif | % dif | p-value |
| 10 | 74.9 | 49.9 | 24.97 | 50 | 0.039 |
| 4 | 35 | 21.7 | 13.28 | 61 | 0.0656 |

Mean absolute values of the measurements of harvest index for the transgenic lines (TR) and control plants (null) in the T1 generation are given in columns 2 and 3, the absolute difference in column 4 and the difference in % in column 5, significance, expressed as a p-value obtained in a t-test, is given in column 6.

The results obtained for the T1 generation were confirmed in the T2 generation; the average increase for harvest index was 13% and an F-test showed this increase was significant (p-value of 0.0447). Furthermore, these T2 data were re-evaluated in a combined analysis with the results for the T1 generation, and the p-value obtained from an F-test showed again that the observed effects were significant (p-value 0.0181).

## Claims

1. Method for improving seed yield of a plant relative to corresponding wild type plants, said method comprising:
- introducing and overexpressing into a plant or plant cell an isolated nucleic acid sequence encoding a GRUBX protein, said nucleic acid sequence being selected from the group:
- a nucleic acid sequence as represented by any one of SEQ ID NO:1, 3, 6;
- a nucleic acid encoding a polypeptide as represented by any one of SEQ ID NO: 2, 4, 7;
- a nucleic acid encoding a polypeptide having at least 80% sequence identity to any one of SEQ ID NO: 2, 4, 7, said polypeptide having ubiquitin-regulatory action within the ubiquitination pathway
- cultivating the plant or plant cell under conditions promoting plant growth.

2. Method according to claim 1, wherein said nucleic acid is derived from a eukaryotic organism, preferably from a plant.

3. Method according to claim 2, wherein said nucleic acid is derived from a dicotyledonous plant, preferably from the family Solanaceae, more preferably from *Nicotiana tabacum.*

4. Method according to claim 2, wherein said nucleic acid is derived from a monocotyledonous plant, preferably from the family Poaceae, more preferably from *Oryza sativa.*

5. Method according to any of claims 1 to 4, wherein expression of said nucleic acid encoding a GRUBX protein is driven by a seed-preferred promoter, preferably a prolamin promoter.

6. Method according to any of claims 1 to 5, wherein said increased seed yield comprises one or more of (i) increased seed biomass, (ii) increased total number of seeds, (iii) increased number of filled seeds, (iv) increased seed size, (v) increased seed volume, (vi) increased harvest index, and (vii) increased Thousand Kernel Weight, all relative to corresponding wild type plants.

7. Use of an isolated nucleic acid sequence encoding a GRUBX protein, said nucleic acid sequence being selected from the group:
- a nucleic acid sequence as represented by any one of SEQ ID NO:1, 3, 6;
- a nucleic acid encoding a polypeptide as represented by any one of SEQ ID NO: 2, 4, 7;
- a nucleic acid encoding a polypeptide having at least 80% sequence identity to any one of SEQ ID NO: 2, 4, 7, said polypeptide having ubiquitin-regulatory action within the ubiquitination pathway, in increasing seed yield of a plant relative to corresponding wild type plants.

## Patentansprüche

1. Verfahren zur Verbesserung des Samenertrags einer Pflanze im Vergleich zu den entsprechenden Wildtyppflanzen, wobei das Verfahren umfasst:
- Einbringen und Überexprimieren einer isolierten Nukleinsäuresequenz, die ein GRUBX-Protein codiert, in eine/einer Pflanze oder Pflanzenzelle, wobei die Nukleinsäuresequenz ausgewählt ist aus der folgenden Gruppe:
- einer Nukleinsäuresequenz, wie sie durch eine der Sequenzen von SEQ ID NO: 1, 3, 6 veranschaulicht wird;
- einer Nukleinsäure, die ein Polypeptid codiert, wie es durch eine der Sequenzen von SEQ ID NO: 2, 4, 7 veranschaulicht wird;
- einer Nukleinsäure, die ein Polypeptid mit mindestens 80% Sequenzidentität zu einer der Sequenzen von SEQ ID NO: 2, 4, 7 codiert, wobei das Polypeptid eine Ubiquitin-regulatorische Wirkung in dem Ubiquitinierungsweg hat;
- Züchten der Pflanze oder Pflanzenzelle unter Bedingungen, die das Pflanzenwachstum fördern.

2. Verfahren nach Anspruch 1, wobei die Nukleinsäure von einem eukaryotischen Organismus, vorzugsweise von einer Pflanze, hergeleitet ist.

3. Verfahren nach Anspruch 2, wobei die Nukleinsäure von einer dikotyledonen Pflanze, vorzugsweise aus der Familie der Solanaceae, stärker bevorzugt von *Nicotiana tabacum* hergeleitet ist.

4. Verfahren nach Anspruch 2, wobei die Nukleinsäure von einer monokotyledonen Pflanze, vorzugsweise aus der Familie der Poaceae, stärker bevorzugt *Oryza sativa* hergeleitet ist.

5. Verfahren nach einem der Ansprüche 1 bis 4, wobei die Expression der Nukleinsäure, die ein GRUBX-Protein codiert, durch einen von Samen bevorzugten Promotor, vorzugsweise einem Prolamin-Promotor getrieben wird.

6. Verfahren nach einem der Ansprüche 1 bis 5, wobei die erhöhte Samenausbeute ein oder mehrere der folgenden Merkmale umfasst: (i) erhöhte Samenbiomasse, (ii) erhöhte Gesamtzahl der Samen, (iii) erhöhte Anzahl gefüllter Samen, (iv) erhöhte Samengröße, (v) erhöhtes Samenvolumen, (vi) erhöhter Ernteindex, und (vii) erhöhtes Tausendkorngewicht, und zwar alle jeweils relativ zu entsprechenden Wildtyp-Pflanzen.

7. Verwendung einer isolierten Nukleinsäuresequenz, die ein GRUBX-Protein codiert, wobei die Nukleinsäuresequenz ausgewählt ist aus der folgenden Gruppe;
- einer Nukleinsäuresequenz, wie sie durch eine der Sequenzen von SEQ ID NO: 1, 3, 6 veranschaulicht wird;
- einer Nukleinsäure, die ein Polypeptid codiert, wie es durch eine der Sequenzen von SEQ ID NO: 2, 4, 7 veranschaulicht wird;
- einer Nukleinsäure, die ein Polypeptid mit mindestens 80% Sequenzidentität zu einer der Sequenzen von SEQ ID NO: 2, 4, 7 codiert, wobei das Polypeptid eine Ubiquitin-regulatorische Wirkung in dem Ubiquitinierungsweg hat, bei der Steigerung des Samenertrags einer Pflanze im Vergleich zu entsprechenden Wildtyppflanzen.

## Revendications

1. Procédé pour améliorer le rendement en graines d'une plante, par rapport à des plantes correspondantes de type sauvage, ledit procédé comprenant :
- l'introduction et la surexpression, dans une plante ou une cellule végétale, d'une séquence d'acide nucléique isolée codant pour une protéine GRUBX, ladite séquence d'acide nucléique étant choisie dans le groupe consistant en :
- une séquence d'acide nucléique telle que représentée par l'une quelconque des séquences SEQ ID N° 1, 3, 6 ;
- un acide nucléique codant pour un polypeptide tel que représenté par l'une quelconque des séquences SEQ ID N° 2, 4, 7 ;
- un acide nucléique codant pour un polypeptide ayant une identité de séquence d'au moins 80 % avec l'une quelconque des séquences SEQ ID N° 2, 4, 7, ledit polypeptide ayant une action régulatrice d'ubiquitine à l'intérieur de la voie d'ubiquitination,
- la culture de la plante ou de la cellule végétale dans des conditions favorisant la croissance de la plante.

2. Procédé selon la revendication 1, dans lequel ledit acide nucléique dérive d'un organisme eucaryote, de préférence une plante.

3. Procédé selon la revendication 2, dans lequel l'acide nucléique dérive d'une plante dicotylédone, de préférence de la famille des Solanaceae, plus particulièrement de *Nicotiana tabacum.*

4. Procédé selon la revendication 2, dans lequel ledit acide nucléique dérive d'une plante monocotylédone, de préférence de la famille des Poaceae, plus particulièrement d'Oryza *sativa.*

5. Procédé selon l'une quelconque des revendications 1 à 4, dans lequel l'expression dudit acide nucléique codant pour une protéine GRUBX est commandée par un promoteur préféré par une graine, de préférence un promoteur de prolamine.

6. Procédé selon l'une quelconque des revendications 1 à 5, dans lequel ladite augmentation du rendement en graines comprend un ou plusieurs de (i) une augmentation de la biomasse des graines, (ii) une augmentation du nombre total de graines, (iii) une augmentation du nombre des graines pleines, (iv) une augmentation du calibre des graines, (v) une augmentation du volume des graines, (vi) une augmentation de l'indice de récolte, et (vii) une augmentation du poids de mille graines, dans tous les cas par rapport aux plantes correspondantes de type sauvage.

7. Utilisation d'une séquence d'acide nucléique isolée codant pour une protéine GRUBX, ladite séquence d'acide nucléique étant choisie dans le groupe consistant en :
- une séquence d'acide nucléique telle que représentée par l'une quelconque des séquences SEQ ID N° 1, 3, 6 ;
- un acide nucléique codant pour un polypeptide tel que représenté par l'une quelconque des séquences SEQ ID N° 2, 4, 7 ;
- un acide nucléique codant pour un polypeptide ayant une identité de séquence d'au moins 80 % avec l'une quelconque des séquences SEQ ID N° 2, 4, 7, ledit polypeptide ayant une action régulatrice d'ubiquitine à l'intérieur de la voie d'ubiquitination, pour augmenter le rendement en graines d'une plante, par rapport à des plantes correspondantes de type sauvage.
